# EUROPEAN PATENT SPECIFICATION

(11) **EP 1 856 274 B1**
(45) Date of publication and mention of the grant of the patent: **27.08.2014**
(21) Application number: 06734812.8
(22) Date of filing: 13.02.2006
(51) Int. Cl.: C12Q 1/34, G01N 33/50

(54) **SYSTEMIC MARKERS FOR ASTHMA AND ANALOGOUS DISEASES**
SYSTEMISCHE MARKER FÜR ASTHMA UND ÄHNLICHE ERKRANKUNGEN
MARQUEURS SYSTÉMIQUES POUR ASTHME ET MALADIES ANALOGUES

(30) Priority: 18.02.2005 US 654169 P
(43) Date of publication of application: 21.11.2007
(73) Proprietor: THE CLEVELAND CLINIC FOUNDATION, Cleveland, OH 44195 (US)
(72) Inventor: HAZEN, Stanley, L., Pepper Pike, Ohio 44124 (US); COMHAIR, Suzy, Concord Township, Ohio 44077 (US)
(74) Representative: Wytenburg, Wilhelmus Johannes
(86) International application number: PCT/US2006/004837
(87) International publication number: WO 2006/091403

(56) References cited:
- US-A1- 2004 054 157
- US-A1- 2004 265 807
- US-B1- 6 583 132
- COMHAIR SUZY A A ET AL: "Superoxide dismutase inactivation in pathophysiology of asthmatic airway remodeling and reactivity." THE AMERICAN JOURNAL OF PATHOLOGY MAR 2005, vol. 166, no. 3, March 2005 (2005-03), pages 663-674, XP002550785 ISSN: 0002-9440
- COMHAIR SUZY A A ET AL: "Correlation of systemic superoxide dismutase deficiency to airflow obstruction in asthma." AMERICAN JOURNAL OF RESPIRATORY AND CRITICAL CARE MEDICINE 1 AUG 2005, vol. 172, no. 3, 5 May 2005 (2005-05-05), - 1 August 2005 (2005-08-01) pages 306-313, XP002550786 ISSN: 1073-449X
- COMHAIR S A ET AL: "Rapid loss of superoxide dismutase activity during antigen-induced asthmatic response" LANCET THE, LANCET LIMITED. LONDON, GB, vol. 355, no. 9204, 19 February 2000 (2000-02-19), page 624, XP004825643 ISSN: 0140-6736
- CHU D ET AL: "Clinical observation of bronchila asthme treated with qingre dingchuan decoction" CHINESE JOURNAL OF INTEGRATIVE MEDICINE,, vol. 1, 1 January 1995 (1995-01-01), pages 281-282, XP001539674 ISSN: 1006-6497
- ANDREADIS ATHENA A ET AL: "Oxidative and nitrosative events in asthma." FREE RADICAL BIOLOGY & MEDICINE 1 AUG 2003, vol. 35, no. 3, 1 August 2003 (2003-08-01) , pages 213-225, XP002551283 ISSN: 0891-5849
- JANSSEN-HEININGER Y ET AL: "SOD INACTIVATION IN ASTHMA BAD NEWS OR NO NEWS?" AMERICAN JOURNAL OF PATHOLOGY, AMERICAN SOCIETY FOR INVESTIGATIVE PATHOLOGY, US, vol. 166, no. 3, 1 March 2005 (2005-03-01) , pages 649-652, XP009049827 ISSN: 0002-9440
- MAK JUDITH C W ET AL: "Systemic oxidative and antioxidative status in Chinese patients with asthma." THE JOURNAL OF ALLERGY AND CLINICAL IMMUNOLOGY AUG 2004, vol. 114, no. 2, August 2004 (2004-08), pages 260-264, XP002551328 ISSN: 0091-6749
- DATABASE MEDLINE [Online] US NATIONAL LIBRARY OF MEDICINE (NLM), BETHESDA, MD, US; January 2003 (2003-01), NADEEM AHMAD ET AL: "Increased oxidative stress and altered levels of antioxidants in asthma." XP002551626 Database accession no. NLM12532099 & THE JOURNAL OF ALLERGY AND CLINICAL IMMUNOLOGY JAN 2003, vol. 111, no. 1, January 2003 (2003-01), pages 72-78, ISSN: 0091-6749

## Description

### BACKGROUND OF THE INVENTION

Asthma is clinically defined as reversible obstructive airway disease. Symptoms of asthma range from chronic cough and wheezing to severe difficulty in breathing and respiratory failure. Acute severe asthma (status asthmaticus) refers to an attack of increased severity that is unresponsive to routine therapy and that, if severe enough, can lead to death.

Asthma is a chronic inflammatory disorder of the airways involving a complex interaction of cells and mediators, most of which result in increased reactive oxygen and nitrogen species (ROS and RNS) in the airways (Gaston, B., et al., 994. Am J Respir Crit Care Med 149(2 Pt 1):538-51; Dweik, R. et al., 2001, Proc Natl Acad Sci U S A 98(5):2622-7; MacPherson, J. C., et al., 2001, J Immunol 166(9):5763-72; Wu, W., et al., 2000, J Clin Invest 105(10):1455-63; Haahtela, T. 1997, Clin Exp Allergy 27(4):351-3.)

There are a number of other chronic inflammatory diseases that, like asthma, are associated with high oxidative and nitrative stress at the disease site. These include sepsis, vasculitis, inflammatory bowel disease, rheumatoid arthritis and cardiovascular disease.

Current regimens for asthma therapy usually maintain normal to near-normal pulmonary function and prevent chronic symptoms. However, in rare cases, asthma is severe or refractory to anti-inflammatory therapies, including corticosteroids (Puddicombe SM, et al., FASEB J 2000, 14:1362-1374).

It is desirable to have additional methods for diagnosing asthma and analogous diseases associated with high oxidative and nitrative stress at the disease site, for determining the severity of asthma in subjects, and for evaluating anti-inflammatory therapies in subjects with asthma and/or an analogous disease associated with increased levels of reactive oxygen and nitrogen species at the disease site.

### SUMMARY OF THE INVENTION

The present invention provides diagnostic methods and markers, prognostic methods and markers, and therapy evaluators for asthma and analogous diseases or inflammatory disorders associated with high oxidative and nitrative stress at the disease site. Examples of such diseases include, but are not limited to, rheumatoid arthritis, vasculitis, inflammatory bowel disease, sepsis, and atherosclerosis.

In one aspect, a diagnostic method for identifying a subject at risk of having asthma and/or an analogous disease is provided. In one embodiment, the method comprises assaying for reduced superoxide dismutase (SOD) activity, preferably reduced total SOD activity, in the serum, or plasma of the subject. Subjects with reduced SOD activity in their plasma, or serum are more likely to have asthma and/or the analogous disease than subjects with normal levels of SOD activity in their serum, or plasma. In another embodiment, the method comprises assaying for elevated levels of one or more oxidatively-modified SOD isoforms or species in the serum or plasma of the subject. Subjects with elevated levels of oxidatively-modified SOD species in their plasma, or serum, are more likely to have asthma and/or the analogous disease than subjects with normal levels of the one or more oxidatively-modified species in their serum, or plasma.

Also provided are prognostic methods for monitoring the progression of asthma and/or an analogous disease in a subject. In one embodiment, the method comprises measuring levels of SOD activity and/or levels of one or more oxidatively-modified SOD species in the serum, or plasma of the subject over time. A decrease in the levels of SOD activity and/or an increase in levels of the one or more oxidiatively-modified SOD species in the serum, or plasma of the subject indicates that the subject's asthma (and/or analogous disease) is worsening. An increase in the levels of SOD activity and/or a decrease in levels of the one or more oxidiatively-modified SOD species in the serum, or plasma of the subject indicates that the subject's asthma (and/or analogous disease) is improving.

Also provided are methods for evaluating the efficacy of anti-inflammatory agents in subjects with asthma and/or an analogous disease associated with high oxidative and/or nitrative stress. The methods comprise determining levels of SOD activity and/or levels of one or more oxidatively-modified SOD species in the serum, or plasma of the subject following treatment with the anti-inflammatory agent. In one embodiment, levels of the systemic marker(s) are then compared to systemic levels of SOD activity and/or levels of the one or more oxidatively-modified SOD species in the subject prior to treatment. In another embodiment, levels of the systemic marker(s) are compared to systemic levels of SOD activity and/or levels of the one or more oxidatively-modified SOD species in control subjects.

Also provided are diagnostic kits for diagnosing asthma and/or an analogous disease associated with high oxidative and nitrative stress at the disease site. The kits provide one or more binding agents that specifically react with an oxidatively-modified form of an SOD species. In certain embodiments the binding agent is an antibody or antibody fragment. Preferably the kit also comprises instructions for using the binding agent to diagnose asthma and/or the analogous disease, for using the binding agent to assess the severity of asthma and/or the analogous disease in the test subject, and/or for using the binding agent to monitor the progression or regression of asthma and/or the analogous disease in the subject.

### BRIEF DESCRIPTION OF THE FIGURES

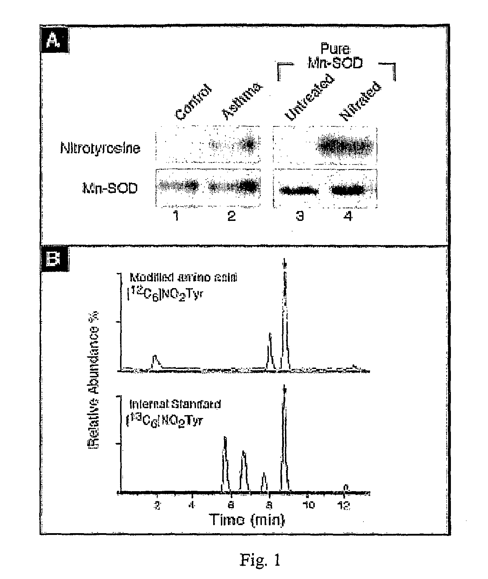

Fig. 1. Nitration of superoxide dismutase (SOD) in asthmatic airway epithelial cells. (a) Lysates from asthmatic or control airway epithelial cells were immunoprecipitated using anti-MnSOD Ab, run on a 4-20% gradient gel, and immunoblotted with anti-nitrotyrosine antibody (upper panel lane 1-2). The lower band confirms equal amount of manganese (Mn)SOD after immunoprecipitation. Pure MnSOD and MnSOD nitrated *in vitro* served as controls (lane 3-4). Experiments were done in triplicate. (b) Protein-bound nitrotyrosine of MnSOD purified from asthmatic airway epithelium was quantified by stable isotope dilution LC-MS interfaced to an HPLX system.

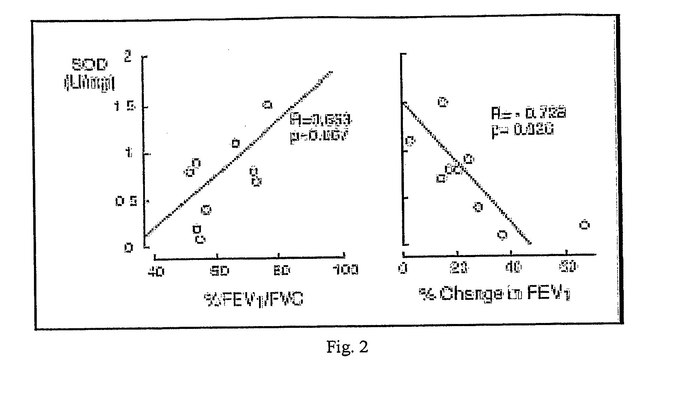

Fig. 2. SOD loss is related to airflow limitation in asthma. Airway epithelial cell SOD activity is inversely correlated to airway response to albuterol (% change in FEV₁) and correlate with %FEV₁/FVC. (b)
Fig. 3. SOD activity in serum of controls (n=20), non-severe (n=75) and severe (n=40) asthmatic individuals. Asthmatic individuals have decreased SOD activity as compared to controls (ANOVA, p= 0.001).
Fig. 4. Analysis of SOD in asthmatic individuals based on airflow limitation. SOD activity is significantly lower in asthmatic individuals with FEV₁ lower than 60% of predicted (ANOVA, p=0.005). %FEV₁<60, n=19; between, n=36;%FEV₁>80, n=59.
Fig. 5. Correlations of serum SOD activity with airflow (%FEV₁, FEV₁/FVC and □ FEV₁). SOD activity is directly correlated with %FEV₁ (R=0.312, p<0.001) and FEV₁/FVC (R=0.296, p<0.001) whereas SOD activity is inversely correlated with hyperresponsiveness, as determined by change in FEV₁ following Beta-agonist (R=-0.334, p=0.001). (controls, n=20; non-severe, n=75; and severe, n=40)
Fig. 6. Analysis of SOD activity corrected for atopy. Individuals with allergies have significant lower levels of serum SOD activity (ANOVA, p=0.027). Interestingly, atopic severe asthmatics show the lowest levels of SOD activity (ANOVA, P=0.042). (controls: non-atopic , n=7; atopic, n=6; non-severe: non-atopic , n=8; atopic, n=54; severe: non-atopic , n=8; atopic, n=30)
Fig. 7. Loss of specific copper zinc (CuZn)SOD activity occurs after protein is exposed to eosinophil peroxidase-generated reactive nitrogen species (RNS), reactive brominating species (RBS) or tyrosyl radicals (•Tyr) *in vitro* (p=0.001).

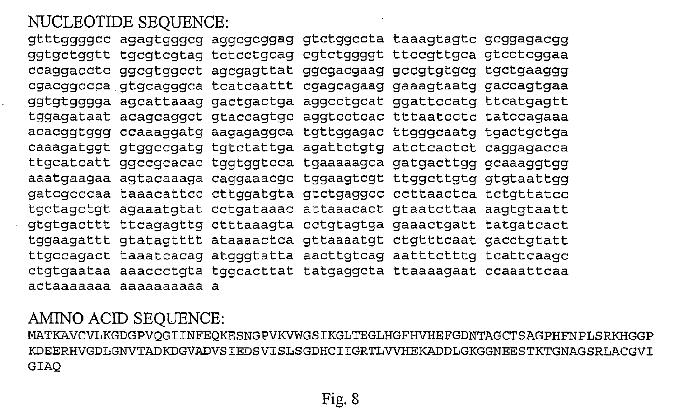

Fig. 8. Nucleotide coding sequence, SEQ ID NO:1 and amino acid sequence, SEQ ID NO: 2, of CuZnSOD, also known as SOD1 (Accession No. NM_000454.)

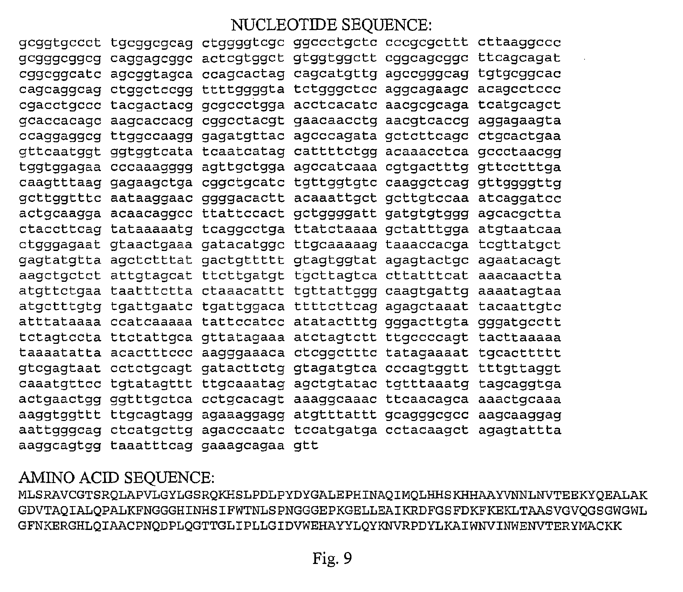

Fig. 9. Nucleotide coding sequence, SEQ ID NO:3 and amino acid sequence, SEQ ID NO: 4, of MnSOD, also known as SOD2 (Accession No. NM_006036.)

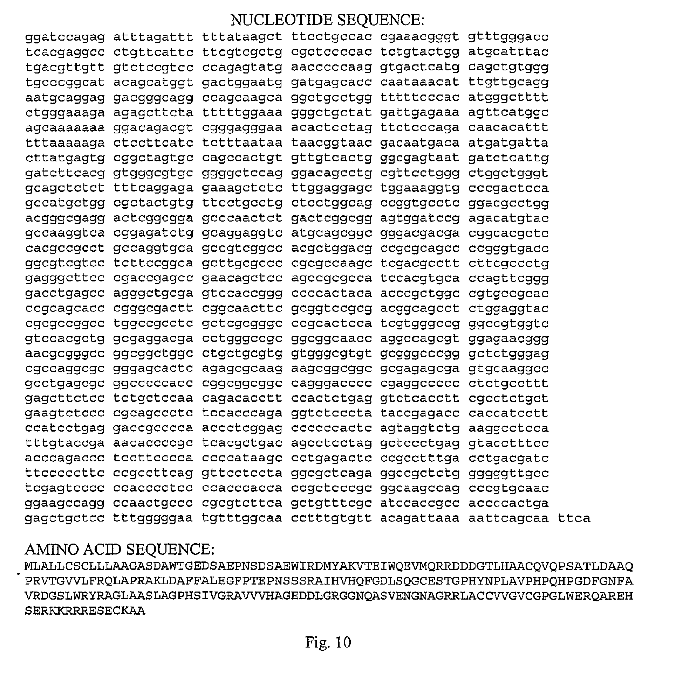

Fig. 10. Nucleotide coding sequence, SEQ ID NO: 5 and amino acid sequence, SEQ ID NO: 6, of extracellular(EC)-OD, also known as SOD3 (Accession No. NM_003102.)

### DETAILED DESCRIPTION OF THE INVENTION

The present invention provides methods and reagents for identifying a subject at risk of having asthma and/or an analogous disease associated with high levels of reactive oxygen and nitrogen species in a subject, for evaluating the severity of asthma in a subject with asthma, for monitoring the effect of anti-inflammatory agents or drugs on subjects with asthma and/or an analogous disease, and for monitoring the progression or regression of asthma and/or the analogous disease in a subject. In certain embodiments, the methods comprise a step which involves determining levels of total superoxide dismutase activity in the serum or plasma of the subject. In certain embodiments, the methods comprise a step which involves determining levels of one or more oxidatively-modified SOD species in the serum, or plasma of the subject. The present invention is based in part on inventors' discovery that SOD activity is reduced in asthmatic individuals and that loss of CuZnSOD activity occurs after the protein is exposed to eosinophil peroxidase-generated reactive nitrogen species (RNS), reactive brominating species (RBS) or tyrosyl radicals.

The present invention will now be described by reference to more detailed embodiments, with occasional reference to the accompanying drawings. This invention may, however, be embodied in different forms and should not be construed as limited to the embodiments set forth herein. Rather these embodiments are provided so that this disclosure will be thorough and complete, and will convey the scope of the invention to those skilled in the art.

Unless otherwise defined, all technical and scientific terms used herein have the same meaning as commonly understood by one of ordinary skill in the art to which this invention belongs. The terminology used in the description of the invention herein is for describing particular embodiments only and is not intended to be limiting of the invention. As used in the description of the invention and the appended claims, the singular forms "a," "an," and "the" are intended to include the plural forms as well, unless the context clearly indicates otherwise.

Unless otherwise indicated, all numbers expressing quantities of ingredients, properties such as molecular weight, reaction conditions, and so forth as used in the specification and claims are to be understood as being modified in all instances by the term "about." Accordingly, unless otherwise indicated, the numerical properties set forth in the following specification and claims are approximations that may vary depending on the desired properties sought to be obtained in embodiments of the present invention. Notwithstanding that the numerical ranges and parameters setting forth the broad scope of the invention are approximations, the numerical values set forth in the specific examples are reported as precisely as possible. Any numerical values, however, inherently contain certain errors necessarily resulting from error found in their respective measurements.

### SUPEROXIDE DISMUTASE (SOD) SPECIES

Superoxide dismutase [EC 1.15.1.1]species include the copper, zinc superoxide dismutase (CuZnSOD) in the cytosol; the manganese superoxide dismutase (MnSOD) in the mitochondria; and the extracellular SOD (EC-SOD).. SODs convert superoxide to hydrogen peroxide (Shull, S., et al., 1991, J Biol Chem 266(36):24398-403; Erzurum, S. C., et al., 1993, J Appl Physiol 75(3):1256-62; Hass, M. A., et al., 1989, J Clin Invest 83(4):1241-6.) while glutathione peroxidases (GPx) (EC 1.11.1.9) removes hydrogen peroxide and organic hydroperoxides in a reaction that consumes the tripeptide glutathione. (Comhair, S. A., et al., 2000, Lancet 355(9204):624.). Despite evidence that localized inactivation of SOD activity occurs within the inflamed asthmatic airways, the relationship of systemic levels of SOD activity to quantitative measures of asthma severity are unknown.

SOD is a first line of defense against oxidant stress and essential for aerobic life. Previous investigations indicate that all three isoforms of SOD contribute to the total SOD activity measured in serum, with reportedly 51% due to CuZnSOD, 13% to MnSOD, and less than 36% to EC-SOD (MacMillan-Crow, L. A., et al., 2001, Free Radic Biol Med 31(12):1603-8. Wu, W., et al., 1999, J Biol Chem 274(36):25933-44.) EC-SOD is found predominantly in the extracellular matrix and to a lesser extent in extracellular fluids ( Macmillan-Crow LA, and Cruthirds DL, Free Radic Res 2001, 34:325-336). More than 90% of EC-SOD is located in the extracellular space bound to heparan sulfate proteoglycans of endothelial cell surfaces and in connective tissue matrix, and significant release to serum requires systemic administration of heparin (Sandstrom, J., P. et al., 1994, J Biol Chem 269(29): 19163-6.).

### SUBJECTS and SAMPLES

As used herein, subject means a mammalian subject. In one embodiment, the subject is a human subject that is suspected of having asthma, e.g., a subject exhibiting one or more symptoms and/or physiologic parameters of asthma, and/or genetically predisposed to asthma. In another embodiment, the subject is a human subject that has been diagnosed as having asthma. In another embodiment, the subject is a human subject exhibiting one or more symptoms of a disease that, like asthma, is associated with high oxidative and/or nitrative stress at the disease site. Examples of such diseases include rheumatoid arthritis, vasculitis, inflammatory bowel disease, sepsis, and to a lesser extent, atherosclerosis.

In an embodiment, the biological sample is plasma. Plasma may be obtained from whole blood samples by centrifugation of anti-coagulated blood. Such process provides a buffy coat of white cell components and a supernatant of the plasma. In another embodiment, the biological sample is serum. Serum may be obtained by centrifugation of whole blood samples that have been collected in tubes that are free of anti-coagulant. The blood is permitted to clot prior to centrifugation. The yellowish-reddish fluid that is obtained by centrifugation is the serum.

The sample may.be pretreated as necessary by dilution in an appropriate buffer solution, heparinized, concentrated if desired, or fractionated by any number of methods. Any of a number of standard aqueous buffer solutions, employing one of a variety of buffers, such as phosphate, Tris, or the like, at physiological pH can be used.

### EMBODIMENTS

In certain embodiments, the methods of the present invention comprise comparing levels of total SOD activity and/or levels of one or more oxidatively-modified SOD species in a sample obtained from the test subject to levels of total SOD activity and/or levels of one or more oxidatively-modified SOD species in samples obtained from subjects lacking the disease, i.e., healthy or normal subjects. Alternatively, levels of total SOD activity and/or levels of one or more oxidatively-modified SOD species may be compared to levels of total SOD activity and/or levels of one or more oxidatively-modified SOD species in corresponding samples which were taken from the test subject for the purpose of determining baseline levels of the diagnostic marker. To establish baseline concentrations in an asthmatic subject, samples are taken at a time when the subject is not exhibiting asthma.

Levels of the present diagnostic markers in the bodily sample of the test subject may be compared to a control value that is derived from levels of the diagnostic marker in comparable bodily samples of control subjects. The control value can be based upon levels of SOD activity, and/or levels of one or more oxidatively-modified SOD species, or both in comparable samples obtained from the general population or from a select population of human subjects. For example, the select population may be comprised of apparently healthy subjects. "Apparently healthy", as used herein, means individuals who have not previously had any signs or symptoms indicating the presence of disease, such as asthma, rheumatoid arthritis, etc. and/or evidence of disease by diagnostic imaging methods. In other words, such individuals, if examined by a medical professional, would be characterized as healthy and free of symptoms of asthma and/or the analogous disease. In an alternative embodiment, levels of the one or more oxidatively-modified SOD species in the test sample may be compared to an internal standard based on levels total SOD and/or levels of unmodified SOD in the subject's bodily sample.

Also provided herein are methods for monitoring over time the status of asthma (and/or the analogous disease) in a subject. In one embodiment, the method comprises determining the levels of one or more of the present diagnostic markers in a biological sample taken from the subject at an initial time and in a corresponding biological sample taken from the subject at a subsequent time. A decrease in levels of SOD activity and/or an increase in levels of the one or more oxidatively-modified SOD species in a biological sample taken at the subsequent time as compared to the initial time indicates that the severity of the subject's asthma (and/or analogous disease) has increased. An increase in levels of SOD activity and/or a decrease in levels of the one or more oxidatively-modified SOD species indicates that the severity of the subject's asthma (and/or analogous disease) has decreased.

In another embodiment, the present invention provides a method for characterizing a subject's response to anti-inflammatory agents therapy directed at stabilizing or regressing asthma and/or an analogous disease associated with increased levels of reactive oxygen and/or nitrogen species at the disease site. Examples of such anti-inflammatory agents include, but are not limited to, steroids and immunomodulating drugs. In one embodiment, the method comprises determining systemic levels of SOD activity and/or one or more oxidatively-modified SOD species in a subject prior to therapy and determining systemic levels of SOD activity and/or one or more oxidatively-modified SOD species in the serum, or plasma of the subject during or following therapy. An increase in levels of SOD activity and/or a decrease in levels of the one or more oxidatively-modified SOD species in the sample taken after or during therapy is indicative of a positive effect of the anti-inflammatory agent in the subject.

In another embodiment, the present invention provides antibodies that are immunospecific for one or more of the oxidatively-modified SOD species that serve as systemic markers in the present methods. In another embodiment, the present invention relates to kits that comprise one or more reagents for assessing SOD activity and/or measuring levels of oxidatively-modified SOD species in biological samples obtained from a test subject. In certain embodiments, the reagents are binding reagents that specifically bind to oxidatively-modified SOD species as opposed to the unmodified SOD species. The present kits also comprise printed materials such as instructions for practicing the present methods, or information useful for assessing the severity of asthma and/or the analogous disease in the test subject. Examples of such information include, but are not limited cut-off values, sensitivities at particular cut-off values, as well as other printed material for characterizing the severity of the disease based upon the outcome of the assay. In some embodiments, such kits may also comprise control reagents.

### BINDING ASSAYS FOR DETERMINING LEVELS OF OXIDIZED SOD SPECIES

Levels of oxidatively-modified SOD and SOD peptide fragments in the biological sample can be determined using binding reagents. The term "binding reagent" and like terms, refers to any compound, composition or molecule capable of specifically or substantially specifically (that is with limited cross-reactivity) binding another compound or molecule, particularly the non oxidatively-modified SOD species. Typically, the binding reagents are antibodies, preferably monoclonal antibodies, or derivatives or analogs thereof, including without limitation: Fv fragments; single chain Fv (scFv) fragments; FAb' fragments; F(ab')2 fragments; polyclonal antibodies and antibody fragments; camelized antibodies and antibody fragments; and multivalent versions of the foregoing. Multivalent binding reagents also may be used, as appropriate, including without limitation: monospecific or bispecific antibodies, such as disulfide stabilized Fv fragments, scFv tandems ((scFv)₂ fragments), diabodies, tribodies or tetrabodies, which typically are covalently linked or otherwise stabilized (i.e., leucine zipper or helix stabilized) scFv fragments. "Binding reagents" also include aptamers, as are described in the art.

Such binding agents specifically or substantially specifically bind to oxidatively modified forms (or fragments thereof) of SOD generated by exposure to the eosinophil peroxidase (EPO)-H₂O₂-NO₂- system, the EPO-H₂O₂-Br⁻ system, HOBr, ONOO⁻, the EPO-H₂O₂-tyrosine system, the myeloperoxidase (MPO) -H₂O₂-NO₂- system, the MPO-H₂O₂-Cl⁻ system, the MPO-H₂O₂-tyrosine system, HOCl, or to copper or iron (+/- H₂O₂) catalyzed oxidation. Such agents have a greater affinity for the oxidatively-modified SOD species than the corresponding native SOD species. In certain embodiments, the oxidatively-modified SOD species (EC-SOD, CuZn SOD or MnSOD) contains one or more of the following: a modified porphyrin prosthetic group, a bromotyrosine, a dibromotyrosine, a nitrotyrosine, a chlorotyrosine, a dichlorotyrosine, a methionine sulfoxide, cysteic acid, sulfenic acid, a carbonyl, a homocitrulline, an amino adipoic acid, cystine, a dihydroxyphenylalanine, a dityrosine, an ortho-tyrosine, and a meta-tyrosine. For example, antibodies immunospecific for nitrotyrosine containing SOD species may be made and labeled using standard procedures and then employed in immunoassays to detect the presence of such nitrotyrosine containing SOD species in the sample. Suitable immunoassays include, by way of example, radioimmunoassays, both solid and liquid phase, fluorescence-linked assays, competitive immunoassays, or enzyme-linked immunosorbent assays. In certain embodiments, the immunoassays are also used to quantify the amount of the oxidized biomolecule that is present in the sample.

Methods of making antigen-specific binding reagents, including antibodies and their derivatives and analogs and aptamers, are well-known in the art. Polyclonal antibodies can be generated by immunization of an animal. Monoclonal antibodies can be prepared according to standard (hybridoma) methodology. Antibody derivatives and analogs, including humanized antibodies can be prepared recombinantly by isolating a DNA fragment from DNA encoding a monoclonal antibody and subcloning the appropriate V regions into an appropriate expression vector according to standard methods. Phage display and aptamer technology is described in the literature and permit in vitro clonal amplification of antigen-specific binding reagents with very low cross-reactivity. Phage display reagents and systems are available commercially, and include the Recombinant Phage Antibody System (RPAS), commercially available from Amersham Pharmacia Biotech, Inc. of Piscataway, N.J. and the pSKAN Phagemid Display System, commercially available from MoBiTec, LLC of Marco Island, Fla. Aptamer technology is described for example and without limitation in U.S. Pat. Nos. 5,270,163, 5,475096, 5,840867 and 6,544,776.

Antibodies raised against the select oxidatively-modified polypeptide species are produced according to established procedures. Generally, an oxidatively-modified SOD or oxidatively-modified SOD peptide fragment is used to immunize a host animal.

Suitable host animals, include, but are not limited to, rabbits, mice, rats, goats, and guinea pigs. Various adjuvants may be used to increase the immunological response in the host animal. The adjuvant used depends, at least in part, on the host species. Such animals produce heterogenous populations of antibody molecules, which are referred to as polyclonal antibodies and which may be derived from the sera of the immunized animals.

Monoclonal antibodies, which are homogenous populations of an antibody that bind to a particular antigen, are obtained from continuous cells lines. Conventional techniques for producing monoclonal antibodies are the hybridoma technique of Kohler and Millstein (Nature 356:495-497 (1975)) and the human B-cell hybridoma technique of Kosbor et al (Immunology Today 4:72 (1983)). Such antibodies may be of any immunoglobulin class including IgG, IgM, IgE, Iga, IgD and any class thereof. Procedures for preparing antibodies against modified amino acids, such as for example, 3-nitrotyrosine are described in Ye, Y. Z., M. Strong, Z. Q. Huang, and J. S. Beckman. 1996. Antibodies that recognize nitrotyrosine. Methods Enzymol. 269:201-209.

### PREPARATION OF BINDING AGENTS

The oxidatively-modified SOD protein or peptide fragment can be used as an immunogen to produce antibodies immunospecific for the oxidiatively-modified SOD protein or peptide fragment. The term "immunospecific" means the antibodies have substantially greater affinity for the oxidiatively-modified SOD species or oxidatively-modified SOD peptide fragment than for other proteins or polypeptides, including the un-modified SOD species or SOD peptide fragment. Such antibodies may include, but are not limited to, polyclonal, monoclonal, chimeric, single chain, and Fab fragments.

Polyclonal antibodies are generated using conventional techniques by administering the oxidatively-modified SOD protein or peptide fragment. to a host animal. Depending on the host species, various adjuvants may be used to increase immunological response. Among adjuvants used in humans, Bacilli-Calmette-Guerin (BCG), and *Corynebacterium parvum.* are especially preferable. Conventional protocols are also used to collect blood from the immunized animals and to isolate the serum and or the IgG fraction from the blood.

For preparation of monoclonal antibodies, conventional hybridoma techniques are used. Such antibodies are produced by continuous cell lines in culture. Suitable techniques for preparing monoclonal antibodies include, but are not limited to, the hybridoma technique, the human B-cell hybridoma technique, and the EBV hybridoma technique.

Various immunoassays may be used for screening to identify antibodies having the desired specificity. These include protocols that involve competitive binding or immunoradiometric assays and typically involve the measurement of complex formation between the respective oxidatively modified SOD polypeptide and the antibody.

The present antibodies may be used to detect the presence of or measure the amount of oxidatively-modified SOD species in a biological sample from the subject. The method comprises contacting a sample taken from the individual with one or more of the present antibodies; and assaying for the formation of a complex between the antibody and a protein or peptide in the sample. For ease of detection, the antibody can be attached to a substrate such as a column, plastic dish, matrix, or membrane, preferably nitrocellulose. The sample may be untreated, subjected to precipitation, fractionation, separation, or purification before combining with the antibody. Interactions between antibodies in the sample and the isolated oxidized SOD protein or peptide are detected by radiometric, colorimetric, or fluorometric means, size-separation, or precipitation. Preferably, detection of the antibody-protein or peptide complex is by addition of a secondary antibody that is coupled to a detectable tag, such as for example, an enzyme, fluorophore, or chromophore. Formation of the complex is indicative of the presence of oxidized SOD protein or peptide fragment in the individual's biological sample.

In certain embodiments, the method employs an enzyme-linked immunosorbent assay (ELISA) or a Western immunoblot procedure.

In certain embodiments of the invention, the binding reagent may be an aptamer. Methods of constructing and determining the binding characteristics of aptamers are well known in the art. For example, such techniques are disclosed in Lorsch and Szostak (In: Combinatorial Libraries: Synthesis, Screening and Application Potential, R. Cortese, ed., Walter de Gruyter Publishing Co., New York, pp. 69-86, 1996) and in U.S. Pat. Nos. 5,582,981, 5,595,877 and 5,637,459. Aptamers may be comprised of DNA or RNA.

In certain embodiments, the starting pool of oligonucleotides (referred to as nucleic acid ligands) used to prepare aptamers will contain a randomized sequence portion flanked by primer sequences that permit the amplification of nucleic acid ligands found to bind to a selected target. Both the randomized portion and the primer hybridization regions of the initial nucleic acid ligand population may be constructed using conventional solid phase techniques. Such techniques are well known in the art (e.g., Froehler, et al., Tet Lett. 27:5575-5578, 1986a; Nucleic Acids Research, 14:5399-5467, 1986b; Nucleosides and Nucleotides, 6:287-291, 1987; Nucleic Acids Research, 16:4831-4839, 1988). For synthesis of the randomized regions, mixtures of nucleotides at the positions where randomization is desired are added during synthesis.

One example of a method of selecting for selecting aptamers of specific binding activity involves use of the SELEX process, disclosed for example in U.S. Pat. No. 5,475,096 and U.S. Pat. No. 5,270,163. SELEX involves selection from a mixture of candidate nucleic acid ligands and step-wise iterations of binding, partitioning and amplification, using the same general selection scheme, to achieve any desired criterion of binding affinity and selectivity. Starting from a mixture of nucleic acid ligands, the method includes: Contacting the mixture with the target under conditions favorable for binding. Partitioning unbound nucleic acid ligands from those nucleic acid ligands that have bound specifically to target analyte. Dissociating the nucleic acid ligand-analyte complexes. Amplifying the nucleic acid ligands dissociated from the nucleic acid ligand-analyte complexes to yield a mixture of nucleic acid ligands that preferentially bind to the analyte. Reiterating the steps of binding, partitioning, dissociating and amplifying through as many cycles as desired to yield highly specific aptamers that bind with high affinity to the target analyte.

In certain embodiments of the invention, one or more labels may be attached to a binding reagent, target biomolecule or other molecule. A number of different labels may be used, such as fluorophores, chromophores, radioisotopes, enzymatic tags, antibodies, bioluminescent, electroluminescent, phosphorescent, affinity labels, nanoparticles, metal nanoparticles, gold nanoparticles, silver nanoparticles, magnetic particles, spin labels or any other type of label known in the art.

Non-limiting examples of affinity labels include an antibody, an antibody fragment, a receptor protein, a hormone, biotin, DNP, and any polypeptide/protein molecule that binds to an affinity label.

SOD activity in a sample may be determined by measuring the rate of reduction of cytochrome c, with one unit (U) of SOD activity defined as the amount of SOD required to inhibit the rate of cytochrome c reduction by 50%, as described in the examples below. Suitable assays may employ the following techniques: UV, VIS, fluorescence spectrophotometry, or chemiluminescence

### EXAMPLES

### EXAMPLE 1

Asthma is commonly diagnosed using physiologic measures, but alterations in airway structure are the defining features of asthma. Damage to airway epithelium, eosinophil infiltration, smooth muscle hyperplasia, thickening and aberrant collagen and protein composition of the basement membrane are well established elements of the asthmatic airway (Bousquet J, et al., Am J Respir Crit Care Med 2000, 161:1720-1745; Davies DE, et al., J Allergy Clin Immunol 2003, 111:215-226). The injury to the bronchial epithelium in asthma is marked by loss of columnar epithelial cells. Extensive loss of cells and denuded basement membrane with few basal cells remaining on the airway surface are noted in severe asthma, but shedding of airway epithelium is present even in clinically mild asthma (Davies DE, et al., J Allergy Clin Immunol 2003, 111:215-226; Busse WW, et al., J Allergy Clin Immunol 2000, 106:1033-1042). Physical loss of epithelial lining cells is considered one proximate cause of the airway hyper-responsiveness to inhaled mediators, and has been speculated to contribute to asthmatic airway remodeling, in particular abnormal collagen synthesis. Evidence from organ culture systems supports the concept of an epithelial-mesenchymal unit in which loss of epithelium leads to mucosal myofibroblast and fibroblast proliferation, and collagen deposition (Davies DE, et al., J Allergy Clin Immunol 2003, 111:215-226; Hocking DC, Chest 2002, 122:275S-278S; Ordonez C, et al., Am J Respir Crit Care Med 2000, 162:2324-2329; Puddicombe SM, et al., FASEB J 2000, 14:1362-1374). Thus, if the epithelial injury and loss could be understood and prevented in asthma, the clinical symptoms of airway hyper-responsiveness and long-term progressive sequelae in the airways, which contribute to fixed airflow limitation, might be prevented.

Several reports have proposed that loss of epithelial cells is due to apoptosis based upon immunostaining for the proteins that regulate apoptosis, or by detection of DNA strand breaks by immunostaining with the TdT-mediated dUTP nick end labeling assay (TUNEL) (Trautmann A, et al., J Allergy Clin Immunol 2001, 108:839-846; Trautmann A, et al., J Allergy Clin Immunol 2002, 109:329-337; Druilhe A, et al., Am J Respir Cell Mol Biol 1998, 19:747-757; Bucchieri F, et al., Am J Respir Cell Mol Biol 2002, 27:179-185; O'Sullivan MP, et al., Am J Respir Cell Mol Biol 2003, 29:3-7). However, not all reports have confirmed increased TUNEL positivity in airways (Druilhe A, et al., Am J Respir Cell Mol Biol 1998, 19:747-757). Furthermore, if airway epithelial cells are undergoing increased cell death, it is unclear whether this is due to an inherent cell defect or a response to the asthmatic airway environment. Although nonspecific events related to increased levels of reactive oxygen and nitrogen species (ROS and RNS) in the asthmatic airway have been postulated to lead to epithelial cell loss, the precise mechanisms of effect are unknown (Comhair SA, Erzurum SC, Am J Physiol 2002, 283:L246-L255; Dweik RA, et al., Proc Natl Acad Sci USA 2001, 98:2622-2627; MacPherson JC, et al., J Immunol 2001, 166:5763-5772; Wu W, et al., J Clin Invest 2000, 105:1455-1463).

We hypothesized that the loss of airway epithelial cells in asthma is due to apoptosis triggered by SOD modification and inactivation. To definitively assess apoptosis in asthma, we evaluated expression and activation of caspases, a family of aspartate directed intracellular proteases required for the terminal stages of apoptosis. Here, we show that caspase- 9, an initiator of apoptosis, and caspase-3, an effector of apoptosis, are activated in asthmatic epithelial cells. Validation that loss of SOD in asthmatic airways can activate the apoptotic pathways in epithelial cells is provided by the complementary approaches of both pharmacological inhibition of SOD activity and molecular silencing of MnSOD mRNA. Physiologic relevance to asthma is supported by a strong and statistically significant inverse relationship between epithelial cell SOD activity and lung function. Finally, SOD inactivation is linked to oxidative modification of MnSOD in vivo via nitration and hydroxylation, protein modifications promoted, respectively, by NO-derived oxidants (peroxynitrite or peroxidase-catalyzed reactions) and hydroxyl radical like oxidants such as those generated by redox active transition metal ions during Fenton and Haber-Weiss oxidation chemistry. Taken together, the present studies provide evidence of ongoing profound oxidative and nitrosative stress in asthmatic airways with downstream consequences of SOD inactivation and airway epithelial cell apoptosis, a defining characteristic of airway remodeling.

### METHODS AND MATERIALS

*Study population.* To evaluate apoptosis in the respiratory system *in vivo,* the study population included 9 healthy nonsmoking individuals and 46 asthmatic individuals. Exclusion criteria for the two groups included age under 18 years or over 65 years, pregnancy, human immunodeficiency virus infection, and history of respiratory infection in the previous 6 weeks, prolonged exposure to second hand smoke at home or at work, exposure to dusty environments or known pulmonary disease producing agents. Asthma was defined based on the National Asthma Education Prevention Program Guidelines, which include: episodic respiratory symptoms, reversible airway obstruction by documentation of variability of FEV₁ and/or FVC by 12% and 200 cc either spontaneously or after 2 puffs inhaled Albuterol, and/or a positive methacholine challenge (Guidelines for the Diagnosis and the Management of Asthma, Expert Panel Report II. 1997:pp 97-4051 National Institutes of Health, Bethesda). None of the subjects had a recent asthma exacerbation, hospitalization, or change in medications for 6 weeks prior to the study. The study was approved by the Institutional Review Boards of the Cleveland Clinic Foundation and the University of Pittsburgh Medical Center, and written informed consent was obtained from all individuals.

*Isolation of bronchial epithelial cells.* Individuals underwent bronchoscopy to obtain samples of human airway epithelial cells (HAEC) with cytology brushings from second- and third -order bronchi with a 1 mm cytology brush (Microvasive, Watertown, MA) as previously described (De Raeve HR, et al., Am J Physiol 1997, 272:L148-L154). The brush sample was immediately placed into sterile media, RPMI 1640 (GIBCO, Rockville, MD) and an aliquot taken for cytology and cell differential determination.

*Cell Culture.* BET1A, a human bronchial epithelial cell line, was cultured in serum-free Lechner and LaVeck medium (LHC-8, Biofluids, Inc., Rockville, MD) with additives 0.33 nM retinoic acid, 2.75 mM epinephrine and the antibiotic combination, 1% penicillin/streptomycin, on plates pre-coated with coating media containing 29 µg/ml collagen (Vitrogen: Collagen Corp., Palo Alto, CA), 10 µg/ml bovine serum albumin (Biofluids, Rockville, MD), and 10 µg/ml fibronectin (Calbiochem, La Jolla, CA) for 5 min. HAEC obtained by bronchial brushing were cultured in serum-free Lechner and LaVeck media (LHC8) on plates pre-coated with coating media (Reddel RR, et al., Cancer Res 1988, 48:1904-1909). To evaluate oxidant stress and antioxidants in apoptotic events, BET-1A cells were stimulated at 70% confluence with SOD inhibitor, 2-Methoxyoestradiol (2-ME) (Sigma, St Louis, MO), or pyrogallol, a superoxide generating compound (J.T. Baker Inc, Phillipsburg, NJ) (Comhair SA, et al., FASEB J 2001, 15:70-78), or hydrogen peroxide (Sigma, St. Louis, MO) in a dose and time dependent manner. 293T cells, a clone of 293 (human embryonic kidney fibroblast cells) that expresses the Simian virus 40 large-T antigen, were maintained in DMEM (Invitrogen) with 10% FCS.

*Antioxidant assays.* Bronchial epithelial cells (BET1A) exposed to 5 □M 2-ME for 30 min to 24 h, or serum of asthmatic individuals, were assayed for glutathione (GSH), glutathione peroxidase (GPx), catalase, and SOD activity. SOD activity was determined by the rate of reduction of cytochrome c, with one unit (U) of SOD activity defined as the amount of SOD required to inhibit the rate of cytochrome c reduction by 50% (Nebot C, et al., Anal Biochem 1993, 214:442-451). The final reaction volume was 3 ml and included 50 mM potassium phosphate buffer, 2 mM cytochrome c, 0.05 mM xanthine, and a 0.1 mM EDTA solution. Xanthine oxidase (Sigma, St Louis, MO) was added at a concentration sufficient to induce a 0.020 per minute change in absorbance at 550 nm. GSH levels were measured as previously described (Comhair SA, et al., Am J Respir Crit Care Med 1999,159:1824-1829).

*Cell Viability and Apoptosis detection.* Cell viability was assessed by bright-field microscopy using a trypan blue dye (0.4 %) exclusion method. The mean survival was determined by examining four different low power fields. Annexin V binding was used to detect apoptotic cells as previously described (Trautmann A, et al., J Allergy Clin Immunol 2001, 108:839-846). Briefly, cells were incubated with 1.0 µg/mL annexin V-FITC and 2.5 µg/mL propidium iodide (BD Biosciences, Palo Alto, CA). The stained cells were analyzed with a FACScan (Becton Dickinson, San Jose, CA), using an argon ion laser at 488 nm and emission recorded at 520 nm with band pass and short pass filters. Gating was done on the forward angle and right angle light scatter only to exclude debris and cell clumps. A minimum of 10,000 cells was measured per condition and all values are expressed as relative fluorescence index (RFI). The RFI was calculated using the ratio of the linearized mean fluorescence of the cell populations, as provided by the CellQuest software (Becton Dickinson, San Jose, CA). Apoptotic cells are identified as the Annexin positive-PI negative fraction.

*Caspase -3- like enzyme activity.* Caspase-3-like activity was measured by a spectrophotometric assay (BD PharMingen, San Diego, CA). This assay measures active caspase-3 binding to fluorogenic Ac-DEVD-AMC substrate and its cleavage to release the fluorescent AMC. AMC fluorescence is quantified by UV spectrofluorometry with an excitation wavelength of 380 nm and an emission wavelength range of 420-460 nm.

The percentage increase in protease activity was determined by comparing the levels of caspase activity in cells recovered from asthmatic versus control subjects.

*Assay for DNA Nicking.* Human bronchial epithelial brushings and bronchial biopsy from controls and asthmatic individuals were evaluated for cell death by the In Situ cell death detection Kit AP, (Boehringer Mannheim, Indianapolis, IN). Following paraffin removal and rehydration for lung tissue, the TdT-mediated dUTP nick end labeling (TUNEL) assay was utilized. Briefly, cell death was visualized by labeling of DNA strand breaks by Terminal deoxynucleotidyl transferase (TdT), which catalyzes polymerization of labeled nucleotides to free 3'-OH DNA ends in a template-independent manner (TUNEL-reaction). The detection of the incorporated fluorescein occurs by an anti-fluorescein antibody conjugated with alkaline phosphatase, which is converted by Vector Red Alkaline Phosphatase Substrate K (Vector Laboratories, Burlingame, CA) or by NBT/BCIP (Roche Diagnostics Co., Indianapolis, IN). Bronchial brushings were smeared on to slides. The positive control cells, A549 treated with DNase I (1.5 mg/ml in 50 mM Tris-HCl, PH 7.5, 1 mg/ml BSA for 30 min), were sedimented on to glass slides using Cytospin (Shandon, Pennsylvania, PA). Cell samples were air dried, fixed with a freshly prepared paraformaldehyde solution (4% in PBS, PH 7.4) for 1 h at room temperature. The slides were evaluated by light microscopy.

*Western Blot Assay.* Airway epithelial cells freshly obtained by bronchoscopic brushing from asthmatic and healthy controls, or BET1A cells, were suspended in buffer (3 mM dithiothreitol, 5 µg/ml aprotinin, 1 µg/ml leupeptin and pepstatin A, 0.1 mM PMSF, 1% NP40 and 40 mM Hepes pH 7.5) and cell lysate prepared by three cycles of freeze/thaw. Total protein was measured by using the Coomassie protein assay (Pierce, Rockford, IL). Whole cell lysate protein was denatured and reduced by treatment with buffer containing 0.05 M Tris (pH 6.8), 1% sodium dodecyl sulfate (SDS), 10% glycerol, 0.00125% bromphenol blue and 0.5%β mercaptoethanol for 3 minutes at 95°C. Total protein was separated by electrophoresis on a 10% SDS-polyacrylamide gel, and then electrophoretically transferred onto nitrocellulose (NitroBind EP4HY315F5, Fisher Scientific, Pittsburgh, PA) or Polyvinylidene Difluoride membrane (Pierce, Rockford, IL) for 1 h at 4°C. Membranes were incubated with blocking buffer [5 % non fat dry milk in TBS (20 mM Tris-HCl (pH 7.0) and 137 mM NaCl) with 0.1% Tween] for 1 h at room temperature to block nonspecific binding and then probed with a primary antibody in blocking buffer overnight at 4°C. Following washing, a peroxidase-conjugated secondary antibody was incubated with the membrane for 1 h at room temperature followed by washes with TBS-0.1% Tween. The detection of signals was performed with an enhanced chemiluminescent system (Amersham Laboratories, Piscataway, NJ). The primary antibodies were anti-mouse monoclonal BAX (Transduction Laboratories, Lexington, KY), PARP, caspase-8 (BD PharMingen, San Diego, CA) and □-actin (Sigma, St. Louis, MO), anti-rabbit polyclonal caspase-3 and caspase-9 (BD PharMingen, San Diego, CA), monoclonal anti-nitrotyrosine antibody (Upstate Biotechnology, Lake Placid, NY) and anti-MnSOD polyclonal antibody (OxisResearch, Portland, OR).

*Immunoprecipitation.* Airway epithelial cells freshly obtained by bronchoscopic brushing from asthmatics and healthy controls were lysed in ice-cold non-reducing lysis buffer (50 mM Tris.HCl (pH 7.4), 150 mM NaCl, 1 mM EDTA, 0.5% Nonidet P-40, 10% glycerol, 1 mM PMSF, 5 µg/ml leupeptin, 10 µg /ml pepstatin A, 20 µg /ml aprotinin and 200 µM NaOV). Immunoglobins were removed by pre-incubating the cell lysis with protein G-Sepharose (Amersham Laboratories, Piscataway, NJ). The supernatant was further incubated with anti-MnSOD antibody (OxisResearch, Portland, OR) followed by protein G-Sepharose incubation. The captured beads were washed and boiled in denaturing, non-reducing buffer. The released proteins were analyzed by western blot as described above. Blots were subsequently evaluated by polyclonal anti-MnSOD antibody to confirm equivalent protein loading.

Two dimentional gel electrophoresis. BET1A cells and HAEC unstimulated or stimulated with 2ME for 24 h were harvested with a lysis buffer composed of 7M urea, 2M thiourea, 4% CHAPS, 1% DTT, 0.5% triton-X-100 and 2% IPG ampholytes (pH 3-10) at room temperature. Samples were sonicated and clarified by centrifugation. Total Protein was measured by using Coomassie protein assay (Pierce, Rockfod, IL). Two dimentional gel electrophoresis was performed with the Isoelectric focussing system (IEP, Bio-Rad; Hercules, CA). 11 cm linear (pH 3-10), immobilized pH gradient strips were used for first dimension. The immobilized pH gradient strips were rehydrated with sample at 50 V for 14 hrs and isoelectric focusing performed by a linear increase to 250 V for 20 min followed by linear increase to 8000 V over 2 hr and 50 min and then held at 8000 V until a total of 43 kVh was reached. For the second dimension, the IPG strips were equilibrated for 15 min in 50 mM Tris-HCl (pH 8.8), 6 M urea, 30% glycerol, 2% SDS, 1% DTT and bromophenol blue, and then 15 min in 50 mM Tris-HCl (pH 8.8), 6M urea, 30% glycerol, 2% SDS, 2% iodoacetamide and bromophenol blue. The strips were embedded in 1% (WT/ vol) agarose on top of 12.5% acrylamide gel containing 4% stacking gel. The second dimension was performed essentially according to Laemmli (Laemmli UK, Cleavage of structural proteins during the assembly of the head of bacteriophage T4. Nature 1970, 227:680-685). After completion of the run, the acrylamide gel was soaked in transfer buffer (20 mM Tris.HCl, 96 mM glycine, 20% methanol) and partially transferred to the polyvinylidene difluoride (PVDF) membrane. The gels were stained with colloidal Coomassie blue (Pierce, Rockford, IL), and western blot was performed as described in method monoclonal anti-nitrotyrosine antibody. Pure and Nitrated MnSOD were used as controls. Briefly, 100 µg of Pure MnSOD (1 mg/ml in 100 mM Tris/HCl, pH 7.5, Sigma, St Louis, MO) was nitrated at room temperature for 30 min by 12 mM tetranitromethane (Sigma, St Louis, MO). Reaction was stropped by adding gel loading buffer with β-mercaptoethanol.

Quantification of purified MnSOD for protein-bound oxidation and nitration. MnSOD was purified by immuno-precipitation. 100 µl protein L gel (Pierce, Rockford, IL) was added to immuno-precipitated MnSOD to deplete IgG. The purity of MnSOD was demonstrated by SDS-PAGE and colloidal blue stain. Purified MnSOD was precipitated by ice-cold acetone, and dried in nitrogen. Protein-bound nitrotyrosine, chlorotyrosine, bromotyrosine, di-tyrosine, o-tyrosine and m-tyrosine were quantified by stable isotope dilution liquid chromatography-tandem mass spectrometry on a triple quadrupole mass spectrometer (API 4000, Applied Biosystems, Foster City, CA) interfaced to a Cohesive Technologies Aria LX Series HPLC multiplexing system (Franklin, MA) using methods as previously described (Zheng L, et al., J Clin Invest 2004, 114:529-541). Briefly, synthetic [¹³C₆]-labeled standards for each analyte were prepared and added to purified MnSOD samples and used as internal standards for quantification of natural abundance analytes. Simultaneously, universally labeled precursor amino acids, [¹³C₉,¹⁵N₁]tyrosine and [¹³C₉,¹⁵N₁]phenylalanine (Cambridge Isotopes Inc., Andover, MA), were added. Following desalting and delipidation, proteins were hydrolyzed under argon atmosphere in methane sulfonic acid, and then samples passed over mini solid-phase C18 extraction columns (Supelclean LC-C18-SPE minicolumn; 3 ml; Supelco, Inc., Bellefone, PA) prior to mass spectrometry analysis. Results were normalized to the content of the precursor amino acid tyrosine (for chlorotyrosine, nitrotyrosine, bromotyrosine and dityrosine) and phenylalanine (for o-tyrosine and m-tyrosine), which were simultaneously monitored within the same injection using characteristic parent daughter ion transitions for each isotopomer of each analyte. Intrapreparative formation of [¹³C₉,¹⁵SN₁]-labeled oxidized tyrosine (chlorotyrosine, nitrotyrosine, dityrosine and bromotyrosine) and phenylalanine (o- and m-tyrosine) species was routinely monitored and negligible (i.e. < 5% of the level of the natural abundance analytes observed) under the conditions employed.

*Transfection of siRNA MnSOD.* MnSOD siRNA was synthesized by Ambion (Austin, TX). The sense and antisense MnSOD siRNA were 5-GGAACAACAGGCCUUAUUCtt-3 (sense) and 5-GAAUAAGGCCUGUUGUUCCtt -3 (antisense). Silencer™ Negative control #1 siRNA (siRNA control) (Ambion, Austin TX) was used as a control. 293T-cells, at 60% confluence in 100 cm plates, were transfected in serum-free medium (DMEM, Invitrogen, Carlsbad CA) by using lipofectamine reagent (Invitrogen, Carlsbad CA) according to the manufacturer's instructions. For the silencing experiments, cells were transfected with 10, 50 and 100 □M of MnSOD siRNA or with 50 nM of Silencer^{TN} Negative Control #1 siRNA. 48 h after transfection, cells were washed, trypsinized and harvested for evaluation of mRNA, protein expression, and caspase-3 activity.

*Northern analysis of MnSOD expression.* Total RNA from 293T-cells was extracted by the GTC [(4 M guanidium thiocyanate, 25 mM sodium citrate pH 7.0), 0.5% sarkosyl, and 0.1 M β-mercaptoethanol]-CsCl gradient method and evaluated by northern analysis using a ³²P-labeled MnSOD (pHMn-SOD4), or as control □-actin cDNA (pHF□A-1), and then subjected to autoradiography.

*Protein identification.* Antinitrotyrosine immunopositive spots were matched with the Coomassie stained 2D gel and identified according to Hanna et al (Hanna SL, et al., Microbiology 2000, 146:2495-2508). The selected protein spots were cored from the gels and placed in a siliconized microcentrifuge tube that had been rinsed with ethanol, water, and ethanol. The gel pieces were washed and destained in 500 µl 50% methanol/5% acetic acid overnight at room temperature before dehydration in 200 µl acetonitrile and complete drying in a vacuum centrifuge. The proteins were reduced by addition of 50µl 10 mM DTT and alkylated by addition of 50 µl 100 mM iodoacetamide. To exchange the buffer, the gel pieces were dehydrated in 200 µl acetonitrile, hydrated in 200µl 100 mM ammonium bicarbonate and dehydrated again with 200 µl acetonitrile. The dehydrated gel pieces were then dried completely in a vacuum centrifuge and rehydrated in 50 µl of 20 ng/µl ice-cold, sequencing-grade modified porcine trypsin (Promega, Madison, WI) for 5 min on ice. Any excess trypsin solution was removed and the digestion carried out overnight at 37 C. The peptides produced in the digest were collected by successive extractions with 50 µl 50 mM ammonium bicarbonate and 50 µl 50% acetonitrile/5% formic acid, combining the extracts in a siliconized 0.6 ml microcentrifuge tube that had been previously rinsed with ethanol, water and ethanol. The total extract was concentrated in a vacuum centrifuge to 20 µl for analysis. The µLC-MS system consisted of a Finnigan LCQ (ThermoQuest) ion-trap mass spectrometer with a Protana nanospray ion source interfaced to a self-packed 8 cmx75 µm i.d. Phenomenex Jupiter 10 µm C18 reverse-phase capillary column; 0·5 µl (2·5%) volumes of peptide extract were injected and the peptides eluted from the column with an acetonitrile/0.1 M acetic acid gradient (2-85% acetonitrile in 30 min) at a flow rate of 0·25 µl min⁻¹. The microspray ion source was operated at 2.8 kV. The digest was analyzed using a full data-dependent acquisition routine in which a full-scan mass spectrum (MS) to determine peptide molecular masses was acquired in one scan and product-ion (MS/MS) spectra to determine amino acid sequence were acquired in the four scans before the cycle repeats. This mode of analysis produces approximately 500 MS/MS spectra of peptides ranging in abundance over several orders of magnitude. Not all MS/MS spectra are derived from peptides. The resulting MS/MS spectra were automatically batch-analysed for each spot using either Ms-fit(http://prospector.ucsf.edu/htmlucsf3.0/msfit.htm) or Mascot (http://www.matrixscience.com).

Immunohistochemical analysis of MIB-1. MIB-1, an antibody directed against recombinant parts of the Ki-67 antigen (Boers JE, et al., Am J Respir Crit Care Med 1998, 157:2000-2006), allows reliable determination of proliferating cells (Boers JE, et al., Am J Respir Crit Care Med 1998, 157:2000-2006). Endobronchial biopsies from asthmatic and control individuals were used for immunostaining. Tissues were fixed in 10% buffered formalin, embedded in paraffin and 5 µm sections were placed on charged slides for immunohistochemistry. Slides were stained with MIB-1 (Immunotech, Marseille, France; dilution 1:25) as previously described.

*Statistical Analysis.* All data are expressed as the mean and standard error of the mean. The comparisons between the three groups were performed using ANOVA. A value of p < 0.05 was considered significant. Linear regression fit of data was performed using GB-STAT™ 6.5 *f*.

### RESULTS

### Clinical characteristics.

Healthy control and asthmatic individuals were similar in terms of gender and age, but varied as to their race [age (yrs): control 37±3, asthma 36±2; gender (M/F): control 4/5, asthma 24/22; Race (African American/Caucasian) control 4/5, asthma 8/38). Asthmatics had positive methacholine challenge and/or evidence of spontaneous airway reactivity [forced vital capacity (FVC % predicted), asthma, 89 ± 3; forced expiratory volume in 1 sec (FEV₁ % predicted), 73 ± 3; %FEV₁/FVC, 71 ± 3]. Numbers of individuals studied for each experiment are stated in the text.

### Increased apoptosis in asthmatic airway epithelial cells.

Airways were examined for histologic changes and apoptosis. Hematoxylin or Hematoxylin Eosin (H&E) staining of lung tissue from controls revealed an epithelium consisting of basal, ciliated, and secretory cells. However, asthmatic epithelium showed marked damage including loss of the bronchial epithelial cells, and thickening of the basement membrane, characteristics of remodeling events. Epithelial cells from asthmatic endobronchial biopsies were strongly TUNEL positive. Evaluation of epithelial cells obtained by bronchial brushing further demonstrated apoptosis, by increased TUNEL staining in asthmatic samples (% TUNEL positive: asthma, 28 ± 3; controls, 0.40 ± 0.16; p <0.05). Polarized airway epithelial cells have a relatively low rate of cell proliferation under healthy conditions, with less than 1 % cell turnover (Boers JE, et al., Am J Respir Crit Care Med 1998, 157:2000-2006). Along with increased cell death, airway epithelial cell proliferation was increased in asthmatic airways as shown by increased immuno-positivity for the proliferation marker MIB-1, detected with an antibody directed against part of the Ki-67 antigen (% MIB-1 positive: asthma, 19.7 ± 2.5; controls, 1.8 ± 0.2).

To verify the apoptotic events in the asthmatic airway epithelial cells, we quantitated caspase-3 cleavage and activation. Caspase-3 activity and cleavage (17 kDa) was detectable in asthmatic epithelium, with asthma showing the highest activity. The increase in caspase-3 activity was related to %FEV₁ of asthmatic patients (r=-0.507, p=0.038). Next we examined activation of the upstream caspase-9, knowing to be required for caspase-3 activation through the mitochondrial pathway and a key cellular target of caspase-3, PARP. Evaluation of the key apoptotic targets in asthma revealed that cleavage fragments of caspase-9 (35 kDa) and PARP (85 kDa) were present in asthmatic epithelial cells, but not in healthy controls. Taken together, the fact that caspase-3 and -9, and PARP cleavage products are found in asthmatic epithelial cells and that caspase-3 activity is increased and correlated with airflow in asthma, we conclude that apoptosis occurs in a disproportionately higher number of asthmatic airway epithelial cells and is related to the pathophysiology of asthma.

### Effects of oxidative stress on airway epithelial cells.

We have previously reported that asthmatic airways have diminished SOD activity with increased loss after antigen challenge (Comhair SA, Erzurum SC, Am J Physiol 2002, 283:L246-L255; De Raeve HR, et al., Am J Physiol 1997, 272:L148-L154). Furthermore, other reports have shown that loss of SOD can initiate apoptosis in some cell types (Siwik DA, et al., Circ Res 1999, 85:147-153), therefore we hypothesized that diminished SOD in airway epithelial cells might be a central event mediating airway epithelial cell apoptosis. To address whether inactivation of SOD and/or increasing reactive oxygen species play a role in airway apoptosis, we treated BET1A cells with pyrogallol, a superoxide producing agent, 2-ME, a SOD activity inhibitor or hydrogen peroxide, in a dose and time dependent manner. 2-ME at 5 µM effectively blocked the activity of SOD by up to 86% at 3 h (p=0.004). However, it did not cause a decrease of SOD protein. Quantitative measures of trypan blue showed decrease in cell viability. The loss of cell viability due to apoptosis in 2ME treated cells was validated by 2 techniques. First, Annexin V staining demonstrated an increase in apoptotic cells as early as 17 h after 2-ME exposure. Second, caspase-3 activity was significantly increased in a time dependent matter. Previous reports have shown that reactive oxygen species lead to apoptosis (Siwik DA, et al., Circ Res 1999, 85:147-153; Macmillan-Crow LA, and Cruthirds DL, Free Radic Res 2001, 34:325-336; Fujimura M, et al., J Neurosci 1999, 19:3414-3422). H₂O₂ and the superoxide producing compound pyrogallol also resulted in loss of SOD activity and increased apoptosis. To verify that loss of SOD may initiate apoposis, we blocked MnSOD RNA using siRNA technique. Loss of MnSOD leads to caspase-3 activation. Taken together, these data support the conclusion that loss of SOD, and specifically MnSOD, is one mechanism of apoptosis in epithelial cells. Of note, MnSOD is central for scavenging intra-mitochrondrial ROS. Loss of MnSOD has been shown to lead to opening of permeability transition pores in the outer mitochondrial membrane and accelerate the release of cytochrome c, triggering apoptosis through activation of caspases (Fujimura M, et al., J Neurosci 1999, 19:3414-3422).

To examine if mitochondria are involved in the apoptotic events in epithelial cells treated with 2 ME, we investigated upstream mediators of caspase-3 activation; i.e. BAX, a death-promoting member of the Bc12 family. The upregulation of BAX levels after 2-ME treatment suggests that oxidative processes and the mitochondria are involved in the activation of caspase-9, -3 and entry into apoptosis. Previous work has shown that oxidative stress decreases intracellular GSH through efflux, and GSH efflux has been identified as a proximal mediator of apoptosis through induction and activation of BAX (Ghibelli L, et al., FASEB J 1998, 12:479-486; Ghibelli L, et al., FASEB J 1999, 13:2031-2036). Here, inhibition of SOD activity also caused rapid depletion of intracellular GSH, consistent with increased intracellular oxidant stress. Thus, our results also link BAX activation to oxidative stress and decreased intracellular GSH *in vitro.* Futhermore BET1A-cells with SOD inhibition by 2ME had an increase in tyrosine nitrated proteins, a marker of peroxynitrite. HAEC exposed to 2ME showed a similar pattern of nitration. Lysates from 2-ME-treated BET1A- cells were evaluated by 2D-gels and corresponding immunopositive proteins were excised from the parent acrylamide gel, digested in-gel with trypsin and tryptic peptides were analyzed with mass spectroscopy. Database searching with the peptide masses identified several proteins (Table 1). Collectively, these data support the notion that apoptosis of airway epithelial cells occurs in response to inhibition of SOD and an increase of reactive oxygen and nitration species. Subsequent decrease in intracellular GSH, which occurs in response to oxidative and nitrative stress, may trigger BAX induction and activation, followed by procaspase-9 and -3 activation.

### Nitration and Oxidation of MnSOD in asthmatic airway epithelial cells

Previous studies indicate that MnSOD is susceptible to oxidative and nitrative modifications, which lead to inactivation (Macmillan-Crow LA, and Cruthirds DL, Free Radic Res 2001, 34:325-336; MacMillan-Crow LA, et al., Free Radic Biol Med 2001, 31:1603-1608; Guo W, et al., Am J Physiol 2003, 285:H1396-H1403; Alvarez B, et al., Free Radic Biol Med 2004, 37:813-822). To investigate whether or not MnSOD protein is modified in asthmatic airways, epithelial cells were recovered during bronchoscopy, the cells lysed, and then MnSOD was immunoprecipitated followed by Western blot analyses using anti-nitrotyrosine antibody. Nitrated MnSOD was identified in the freshly obtained asthmatic airway epithelial cells. To investigate the degree of nitration and oxidation, MnSOD was purified by immunoprecipitation and molecular markers of multiple distinct oxidative pathways were quantified by stable isotope dilution tandem mass spectrometry (Table 2). Interestingly, the oxidation of phenylalanine to m-Tyr and o-Tyr such as via exposure to hydroxyl radical-like oxidants, chlorination of tyrosine (a specific molecular marker for myeloperoxidase-catalyzed halogenation), and oxidative cross-linking of tyrosine as monitored by dityrosine (a product of tyrosyl radical) were the dominant modifications noted. This pattern of oxidative modification is consistent with MnSOD exposure to both Fenton / Haber-Weiss reaction mechanisms i.e. redox active transition metal ion catalyzed oxidation and myeloperoxidase-catalyzed oxidation, even in airways of mild asthmatics. Consistent with our immunodetection studies, nitration of tyrosine was also present in MnSOD recovered from asthmatic airway epithelial cells, indicating exposure to nitrating oxidants such as peroxynitrite/peroxycarboxynitrite or peroxidase-mediated reactive nitrogen species (MacPherson JC, et al., J Immunol 2001, 166:5763-5772; Guo FH, et al., J Clin Invest 1997, 100:829-838). While the oxidative modifications monitored only represent a subfraction of total oxidative insults experienced by epithelial cell MnSOD within asthmatic airways, the quantification of this diverse array of distinct oxidative modifications provides insight into the potential degree of SOD functional impairment from oxidative processes. Given that there are 10 tyrosine residues per monomer and 4 monomers form an active MnSOD tetramer, if modification of only one tyrosine per MnSOD tetramer is sufficient to affect activity, then the observed cumulative modification burden of 1.13-1.73 mmol/mol tyrosine in isolated MnSOD predicts up to a 6% loss of MnSOD activity in these mild asthmatics. It is interesting to speculate that loss of activity may be greater in asthma exacerbation and in severe asthma conditions in which generation of reactive oxygen and nitrogen species is greatly increased (MacPherson JC, et al., J Immunol 2001, 166:5763-5772; Wu W, et al., J Clin Invest 2000, 105:1455-1463; Calhoun WJ, et al., Am Rev Respir Dis 1992, 145:317-325).

### Relation of SOD to clinical features of asthma.

Loss of airway epithelial cells has been postulated to be a contributing mechanism to the airway hyper-responsiveness of asthma (Smith LJ, et al., Free Radic Biol Med 1997, 22:1301-1307). On the basis that reduction of SOD activity is directly linked to apoptotic death of bronchial epithelial cells, we hypothesized that diminished SOD activity might be related to physiologic parameters of asthma *in vivo.* To test this, we evaluated airway activity of antioxidant enzymes in 9 asthmatics in relation to airflow and responsiveness to inhaled bronchodilator. SOD activity in airway epithelial cells of asthmatics correlated with %FEV₁/FVC, and demonstrated significant inverse correlation with airway reactivity as determined by % change in FEV₁ after bronchodilator, although FEV₁ itself did not correlate with SOD activity. Interestingly, SOD activity was the only antioxidant enzyme that correlated with pulmonary function of asthmatic individuals (Table 3).

### DISCUSSION

Recent progress has revealed asthma as a chronic inflammatory disease (Dweik RA, et al., Proc Natl Acad Sci USA 2001, 98:2622-2627; MacPherson JC, et al., J Immunol 2001, 166:5763-5772; Wu W, et al., J Clin Invest 2000, 105:1455-1463; Barnes PJ, N Engl J Med 2000, 343:269-280; Drazen JM, et al., N Engl J Med 1999, 340:197-206; Kaminsky DA, et al., J Allergy Clin Immunol 1999, 104:747-754). Current understanding suggests that inflammation leads to remodeling events in the airway, which are often progressive and contributory to severe morbidity and refractoriness to treatment. However the specific mechanisms by which inflammation leads to asthmatic airway remodeling are unclear (Bousquet J, et al., Am J Respir Crit Care Med 2000, 161:1720-1745; Davies DE, et al., J Allergy Clin Immunol 2003, 111:215-226; Busse WW, et al., J Allergy Clin Immunol 2000, 106:1033-1042; Kelly EA, et al., Am J Respir Crit Care Med 2000, 162:1157-1161). Here, we reveal apoptosis as a mechanism for airway epithelial cell loss, a hallmark of remodeling in asthma, and identify loss of catalytically active SOD as an initiating event for entry into programmed cell-death. Apoptosis in asthmatic airway epithelial cells was confirmed from three lines of evidence. First, immunostaining of endobronchial or brush biopsies reveals a striking increase in TUNEL-positive bronchial epithelial cells in asthma as compared to healthy nonsmoking controls. Previous studies have suggested epithelial cell apoptosis in the airways through observation of TUNEL positive cells (Trautmann A, et al., J Allergy Clin Immunol 2002, 109:329-337) and caspase-3 and PARP immunostaining in biopsies of asthmatic individuals (Bucchieri F, et al., Am J Respir Cell Mol Biol 2002, 27:179-185). However, Druilhe et al. (Druilhe A, et al., Am J Respir Cell Mol Biol 1998, 19:747-757) noted a failure to detect differences in the number of TUNEL-positive bronchial epithelial cells between control and asthmatic airway endobronchial biopsies, perhaps due to detachment and loss of many of the apoptotic epithelial cells into the lumen during the process of biopsy. Others have indicated that the loss of epithelium in asthmatic biopsies may be an artifact of sampling (Ordonez C, et al., Am J Respir Crit Care Med 2000, 162:2324-2329).

In this study, cells obtained by gentle brushing of the airway from asthmatic and healthy controls show an increase in TUNEL positive cells in asthmatic biopsies, while only rare cells are TUNEL positive in healthy control brushings. The marked increase of proliferative cells in asthma as determined by increased MIB-1, together with a previous report of increased expression of epidermal growth factor receptor (Puddicombe SM, et al., FASEB J 2000, 14:1362-1374), provides conclusive evidence for apoptosis, as enhanced proliferation of cells is a repair mechanism which occurs in association with accelerated apoptosis and loss of cells (Davies DE, et al., J Allergy Clin Immunol 2003, 111:215-226). Ongoing repair also substantiates that epithelial damage and shedding is in progress *in vivo.* Finally, the terminal stages of apoptosis require the activation of caspases by proteolytic cleavage, which then proteolytically cleave cellular proteins, including PARP. Hence, the detection of the cleaved form of caspase-3 and the increased activity is undeniable evidence of ongoing apoptosis in asthmatic airway epithelial cells.

Here, we show that loss of SOD activity, specific inhibition of MnSOD, and/or increased production of superoxide leads to increased levels of BAX, cleavage and activation of caspase-3 and changes in the redox state of the cells. Previously, we have shown that airway epithelial cells exposed to oxidative stress rapidly shunt out glutathione, resulting in increased extracellular, but transient depletion of intracellular glutathione (Comhair SA, et al., FASEB J 2001, 15:70-78). Interestingly, efflux of glutathione reproducibly activates BAX and cytochrome c release in epithelial cells *in vitro* and is one established mechanism for induction of apoptosis (Ghibelli L, et al., FASEB J 1998, 12:479-486; Ghibelli L, et al., FASEB J 1999, 13:2031-2036; Jungas T, et al., J Biol Chem 2002, 277:27912-27918). In support of increased transcellular glutathione fluctuation in the upstream events leading to epithelial cell apoptosis in asthma, glutathione levels are higher than normal in the asthmatic airway lining fluid, indicating increased efflux from epithelial cells *in vivo* (Kelly EA, et al., Am J Respir Crit Care Med 2000, 162:1157-1161; Kelly FJ, et al., Lancet 1999, 354:482-483; Meerschaert J, et al., Am J Respir Crit Care Med 1999, 159:619-625; Smith LJ, et al., Am Rev Respir Dis 1993, 147:1461-1464).

Here, evidence for reactive oxygen and nitrogen species involvement in airway epithelial cell apoptosis in asthma include the finding of increased nitrotyrosine in epithelial cells after inhibition of SOD *in vitro,* and in airway epithelial cells *in vivo* in asthma in other studies (Dweik RA, et al., Proc Natl Acad Sci USA 2001, 98:2622-2627; Comhair SA, et al., FASEB J 2001, 15:70-78; Saleh D, et al., FASEB J 1998, 12:929-937). Here, we provide quantitative data on MnSOD oxidation and nitration in human asthmatic lungs. Between 5 and 7% of MnSOD recovered from asthmatic airway epithelial cells possess at least 1 oxidative modification, with the majority of modifications related to Fenton-Haber Weiss reaction chemistry and/or peroxidase-catalyzed oxidation. Although not evaluated in this study, Alvarez et al (Alvarez B, et al., Free Radic Biol Med 2004, 37:813-822) recently showed that peroxynitrite causes oxidative modifications of the CuZnSOD and loss of activity through formation of histidinyl radicals. Hence, oxidative inactivation of CuZn SOD may also contribute to the loss of total SOD activity noted in asthma (De Raeve HR, et al., Am J Physiol 1997, 272:L148-L154; Smith LJ, et al., Free Radic Biol Med 1997, 22:1301-1307). The loss of SOD activity likely reflects the increased oxidative and nitrative stress in the asthmatic airway, and may serve as a marker of asthma severity. Here, reactivity measured as the change in FEV₁ after bronchodilator confirms the association of airway hyper-reactivity to SOD activity. Based upon this study and others, we propose that loss of SOD activity in asthma occurs, in part, as a consequence of MnSOD protein modifications in the oxidative and nitric oxide rich environment of the asthmatic airway, and that SOD inactivation and oxidant stress trigger apoptosis and loss of airway epithelial cells, which contributes significantly to airway remodeling and hyper-reactivity of asthma.

**Table 1: Identification of nitrated proteins in airway epithelial cells exposed to the SOD inhibitor, 2-Methoxyoestradiol**

| **Protein** | **pI** | **Mol Wt. kDa** | **Accession No** |
|---|---|---|---|
| Fascin | 6.8 | 55 | 2498357 |
| Dihydrolipoamide | 7.6 | 55 | 66123 |
| Alpha enolase | 6.9 | 47 | 119339 |
| Voltage dependent anion channel 2 | 6.8 | 30 | 31890058 |
| Ran full-length protein chain | 9.4 | 21 | 5107637 |
| Citrate synthase | 8.4 | 51 | 14603295 |
| Actin | 5.8 | 40 | 16359158 |
| Phosphoglycerate kinase | 8.3 | 44 | 2144428 |
| Fructose-biphosphate | 8.3 | 39 | 68183 |
| aldolase | | | |
| 1-lactate dehydrogenase 8.4 | | 36 | 65922 |
| Glyceraldehydes-3-phosphate dehydrogenase | 8.5 | 36 | 625203 |
| Voltage dependent anion selective channel | 8.6 | 30 | 130683 |
| protein 1 | | | |
| Histone h3/b | 11.2 | 15 | 18202621 |
| Histone h2b | 10.3 | 13 | 7381193 |
| Peptidylprolyl isomerase | 7.6 | 18 | 118102 |

Nitrated proteins found on 2D gel were identified by peptide mass mapping using product-ion (MS/MS) spectra.

**Table 2: Oxidative modifications in MnSOD from asthmatic airway epithelial cells.**

| **NO₂Y/Y** | **BrY/Y** | **ClY/Y** | **mY/Phe** | **oY/Phe** | **DiY/Y** | **Total** |
|---|---|---|---|---|---|---|
| 0.10-0.13 | 0.02-0.04 | 0.11-0.47 | 0.03-0.24 | 0.16-0.55 | 0.31-0.71 | 1.13-1.73 |

Data presented represent ranges in values observed in MnSOD isolated from epithelial cell brushings from mild asthmatic subjects. Results are normalized to the content of the precursor amino acid (mmol oxidation product/mol precursor tyrosine or phenylalanine), which is monitored within the same injection.. All data are representative of 4 asthmatic individuals. Y, tyrosine; NO₂Y, Nitrotyrosine; ClY, Chlorotyrosine; BrY, Bromotyrosine, DiY, Dityrosine; oY, o-tyrosine; mY, m-tyrosine, Phe, phenylalanine.

**Table 3: Correlation of lung functions with asthmatic airway epithelial cell antioxidant enzymes**

| Lung Functions | SOD | GPx | Catalase |
|---|---|---|---|
| | | | |
| %FEV₁/FVC | R=0.663 | R= 0.088 | R= - 0.400 |
| | p= 0.067 | p= 0.821 | p= 0.286 |
| %change in FEV₁ | R= -0.728 | R= 0.326 | R= 0.383 |
| | p= 0.026 | p= 0.391 | p= 0.309 |

| | | | |
|---|---|---|---|
| FEV₁: forced expiratory volume in 1 sec; FVC: forced vital capacity | | | |

### EXAMPLE 2:

### INTRODUCTION

We undertook a study with cross-sectional samples obtained throughout the US and England to assess systemic antioxidant enzyme activities for SOD and the GPx/glutathione system, and the relationship between antioxidants, asthma severity, airflow limitation and hyperresponsiveness. Potential mechanisms of SOD inactivation were examined in model systems, while in parallel, serum enzyme activity levels in subjects were related to circulating levels of molecular markers of distinct oxidative pathways known to be increased in severe asthma, such as those produced by eosinophil peroxidase-generated reactive brominating species, nitric oxide-derived oxidants, and tyrosyl radical (MacPherson, J.C., et al., J Immunol 166(9):5763-72; Wu, W., et al., J Clinc Invest 105(10):1455-63; Andreadis, A.A., et al., Free Radic Biol Med 35(3):213-25).

### METHODS

### Study population

To evaluate SOD in serum, the study population included 135 individuals comprised of 20 healthy nonsmoking individuals and 115 asthmatic individuals (75 non-severe and 40 severe asthmatics). All samples were collected by investigators in the NHLBI Severe Asthma Research program (SARP). Severe asthma was based on the definition used by the proceedings of the American Thoracic Society Workshop on Refractory Asthma (2000. Proceedings of the ATS Workshop on Refractory Asthma. Current Understanding, Recommendations and Unanswered Questions. Am J Respir Crit Care Med 162(6):2341-2351), with major and minor characteristics. Defining major characteristics include (1) treatment with continuous or near continuous oral corticosteroids, and/or (2) high dose inhaled corticosteroids. The minor criteria are as follow: (1) Daily treatment with controller medication in addition to inhaled corticosteroids; (2) use of short-acting □-agonist on a daily or near daily basis; (3) Persistent airway obstruction [FEV₁>80% predicted and diurnal peak expiratory flow (PEF) variability >20%]; (4) one or more urgent care visits for asthma per year; (5) Three or more oral corticosteroid bursts per year; (6) prompt deterioration with reduction in oral or inhaled corticosteroid dose; (7) Near-fatal asthma event in the past.

Subjects enrolled in SARP were classified as healthy controls, non-severe or severe asthma. Subjects met criteria for severe asthma with at least 1 major and at least 2 minor criteria. Inclusion criteria for control subjects were (1) lack of cardiopulmonary symptoms, (2) normal baseline spirometry, and (3) a negative methacholine challenge test (defined as less than 20% decline in FEV₁ with the maximum dose of methacholine). Exclusion from SARP enrollment for asthmatic and control subjects included current smoking history, or smoking history within one year, former smokers with greater then 5 pack-year total history, pregnancy and human immunodeficiency virus infection. The study was approved by all SARP centers Institutional Review Boards and written informed consent was obtained from all individuals.

### Procedures to characterize volunteers

Lung function. Spirometry was performed on an automated spirometer consistent with American Thoracic Society standards. The FVC, FEV₁, and FEV₁ to FVC ratio were collected for each of three efforts before and after the administration of two albuterol puffs via Aerochamber. Reference equations for spirometry are those of National Health and Nutrition Examination Survey (NHANES III).

Atopy. All volunteers underwent skin testing with the Multi-Test II (Lincoln Diagnostics, Inc). Allergy skin testing was performed with the following antigens: cat allergen, dog hair, D. Pteryn, D. Farinae, cockroach, tree mix, ragweed mix, common weed mix, molds including Atlternaria, Aspergillus, and Cladosporium, normal saline as negative control, and histamine as positive control. Allergens were obtained from Hollstier Stier, Spokane, Washington and tested to make sure they are free of lipoplysaccharide (LPS) contamination. Fifteen minutes after the application of the allergen, a study coordinator assessed redness and/or swelling at the site. Significant tests were those in which the application of an allergen produces a wheal with diameter of 3 mm or more than the negative control or a flare with diameter of 10 mm or more. Allergy or atopy was defined as two or more positive skin tests in the presence of positive histamine reaction. Allergy skin testing was done once on each subject during the study.

Airway reactivity. Methacholine challenge testing was performed in all volunteers. However, patients with a baseline %FEV₁ lower than 55% did not undergo a methacholine challenge. The degree of airway narrowing was measured by using the forced expiratory spirometry, particularly FEV₁. Increasing concentrations of methacholine were delivered until FEV₁ fell at least 20% when compared to a control (postdiluent) level. The measure used to compare the sensitivity of one individual to another was PC₂₀, the first provocative concentration that caused a 20% fall in FEV₁.

### Extracellular Glutathione Peroxidase protein (eGPx).

eGPx protein was measured by enzyme-linked immunosorbent assay (ELISA) (Calbiochem, La Jolla, CA). This method is based on a sandwich-type immunoassay, and is specific for eGPx. The eGPx protein concentration present in serum was obtained using a 4-parameter curve fit generated from known standard concentrations of human eGPx.

### Total GSH (GSH+GSSG).

GSH levels in serum were measured by standard methods as previously described (26). In brief, total glutathione levels were determined by mixing equal volumes of serum with 10 mM 5,5'-dithiobis-2-nitrobenzoic acid (DTNB) in 100 mM potassium phosphate, pH 7.5, which contained 17.5 µM EDTA. An aliquot (50µl) of the solution was added to a cuvette containing 0.5 U of glutathione disulfide reductase (Sigma type III, Sigma Chemical, St. Louis, MO) in 100 mM potassium phosphate and 5 mM EDTA, pH 7.5. After 1 minute, the reaction was initiated with 220 nmol of NADPH in a final reaction volume of 1 ml. The rate of reduction of DTNB was recorded continuously at 412 nm by a spectrophotometer with a Kinetics/Time feature (Beckman DU-640, Beckman Instruments, Inc. Fullerton, CA).

### Glutathione Peroxidase (GPx) activity

Total glutathione peroxidase activity was determined spectrophotometrically in serum. Serum was incubated in the presence of 0.1 mM sodium azide, 1 U/ml glutathione reductase, 0.1 mM glutathione and 0.12 mM reduced□-nicotinamide adenine dinucleotide phosphate (□-NADPH), 0.016 mM dithiothreitol, 0.38 mM EDTA and 50 mM sodium phosphate (pH 7.0) for 2 minutes at 25°C. The reaction was initiated by the addition of 0.2 mM hydrogen peroxide. The decrease in absorbance at 340 nm over 3 minutes as NADPH is converted to NADP is proportional to the GPx activity. One unit of activity is defined as the activity that catalyzed the oxidation of 1 nmol NADPH/min using an extinction molar coefficient of 6.22x10⁶ M⁻¹ cm⁻¹ for NADPH (1).

### SOD activity assay.

SOD activity was determined by the rate of reduction of cytochrome c, with one unit (U) of SOD activity defined as the amount of SOD required to inhibit the rate of cytochrome c reduction by 50% (Nebot, C., eta l., Anal Biochem 214(2):422-51). The final reaction volume was 3 ml and included 50 mM potassium phosphate buffer, 2 mM cytochrome c, 0.05 mM xanthine, and a 0.1 mM EDTA solution. Xanthine oxidase (Sigma, St Louis, MO) was added at a concentration sufficient to induce a 0.020 per minute change in absorbance at 550 nm.

### Sample preparation and mass spectrometry.

**Protein-bound** 3-nitrotyrosine, 3-bromotyrosine, and o,o'-dityrosine were determined by stable isotope dilution liquid chromatography-tandem mass spectrometry on a triple quadrupole mass spectrometer (Quattro II Ultima, Micromass, Inc.) interfaced to a Cohesive Technologies Aria LX Series HPLC multiplexing system (Franklin, MA) ( Brennan, M.L., et al., J Biol Chem 277(20):17415-27; Eiserich, J.P., et al., Science 296(5577):2391-4). Briefly, aliquots of plasma (200 µg protein) were desalted and delipidated using a single phase extraction mixture comprised of aqueous sample:methanol:water-washed diethyl ether (1:3:7; v/v/v), the protein pellet supplemented with isotope labeled internal standards ([¹³C₉,¹⁵N₁]tyrosine, 3-bromo[¹³C₆]tyrosine, 3-nitro[¹³C₆]tyrosine, and o,o' di-[¹³C₁₂]tyrosine for quantification of the respective parent and oxidized amino acids), subjected to acid hydrolysis with methane sulfonic acid, passed over solid-phase C18 extraction columns (Supelclean LC-C18-SPE minicolumn; 3 ml; Supelco, Inc., Bellefone, PA), and then analyzed by injection onto reverse phase analytic HPLC columns interfaced with the mass spectrometer using multiple reaction monitoring mode for characteristic parent/daughter ion transitions for each analyte and its appropriate isotopomers (Zheng, L., et al., J Clin Invest 114(4):529-41). Results are normalized to the content of the precursor amino acid tyrosine, which was monitored within the same injection. Intrapreparative formation of [¹³C₉¹⁵N]tyrosine-derived 3-bromotyrosine, o,o'-dityrosine and 3-nitrotyrosine were routinely monitored for in all analyses and shown to be negligible under the sample preparation conditions employed (i.e. < 5% of the level of the natural abundance product observed).

### Superoxide dismutase treatment with eosinophil peroxidase-derived reactive species.

CuZnSOD (Calbiochem, La Jolla, CA) with specific activity of 3.78 U/µg protein was exposed to the eosinophil peroxidase (120 nM final)/H₂O₂ (100 µM final) system in the presence of either sodium bromide (100 µM final), nitrite (100 µM final) or tyrosine (100 µM final) for 30min at 37 °C. Reactions were performed in potassium phosphate buffer (15 mM, pH 7.0) supplemented with 200 µM diethylenetriaminepentaacetic acid. Reactions were quenched by addition of methionine (100 µM) and snap freezing in liquid nitrogen.

### Statistical Analysis.

Data were summarized using the mean and its standard error (SEM). Group comparisons were performed with analysis of variance (ANOVA), and tests were performed at individual significance levels of □=0.05 (i.e., p<0.05 was considered significant). Associations between SOD activity and each of age, gender, and medication were assessed using linear models and ANOVA, and these factors were included as covariates in linear models for the group comparisons. All tests and model-fitting were performed with the R statistical language, version 1.9.0
(R=Development Core Team (2004). R: A language and environment for statistical computing. R Foundation for Statistical Computing, Vienna, Austria. ISBN 3-900051-00-3, URL http://www.R-project.org.)

### RESULTS

### Subject Characteristics

A total of 135 patients were enrolled in the study. Baseline characteristics are shown in Table 1. On average, healthy controls and non-severe asthmatics were younger than severe asthmatics (p<0.05) (Table 4). As expected, lung functions were lower in severe asthmatics than in non-severe or healthy controls.

### Evaluation of Antioxidants

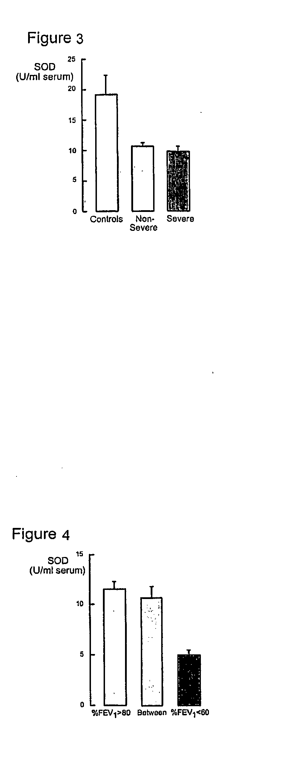

*Analysis of antioxidants by severity.* To investigate if oxidative stress in asthmatic airways influences systemic antioxidants, Glutathione Peroxidase activity (GPx), extracellular Glutathione Peroxidase protein (eGPx), total glutathione (GSH), and total SOD activity were measured in serum. Total SOD activity was significantly different in the 3 groups (p=0.001) (Figure 3). Serum GSH levels tended to be lower in non-severe asthmatics (p=0.084), but higher in severe asthmatics than controls [(GSH (µM): control, 1.69±0.19; non-severe, 1.35 ±0.09; severe, 2.30±0.73; p=0.193]. GPx activity and eGPx protein were not significantly different in the 3 groups (p>0.05).

### Analysis of antioxidants by airflow limitation in asthma.

Antioxidants were also evaluated on the basis of %FEV₁ measurements (%FEV₁>80, FEV₁ between 60 and 80, and %FEV₁<60)(Figures 4). SOD activity was significantly related to airflow limitation (ANOVA p=0.005) (Figure 4). GPx-activity and eGPx protein in the asthmatic group were similar among the groups. GSH was significantly increased in asthmatic patients with severe airflow limitation (%FEV₁<60) relative to either intermediate (%FEV₁, 60-80) or mild (%FEV₁ >80) disease (T-test, p=0.024).

### Age Adjusted group effect within the asthma group

The mean age in the severe asthma group was higher than that of healthy controls. Therefore, difference among groups was also tested with an ANOVA model that adjusted for age. SOD activity (p=0.004) remained significantly different among the asthmatic and control groups when adjusted for age.

### Multiple Linear Regression Analysis

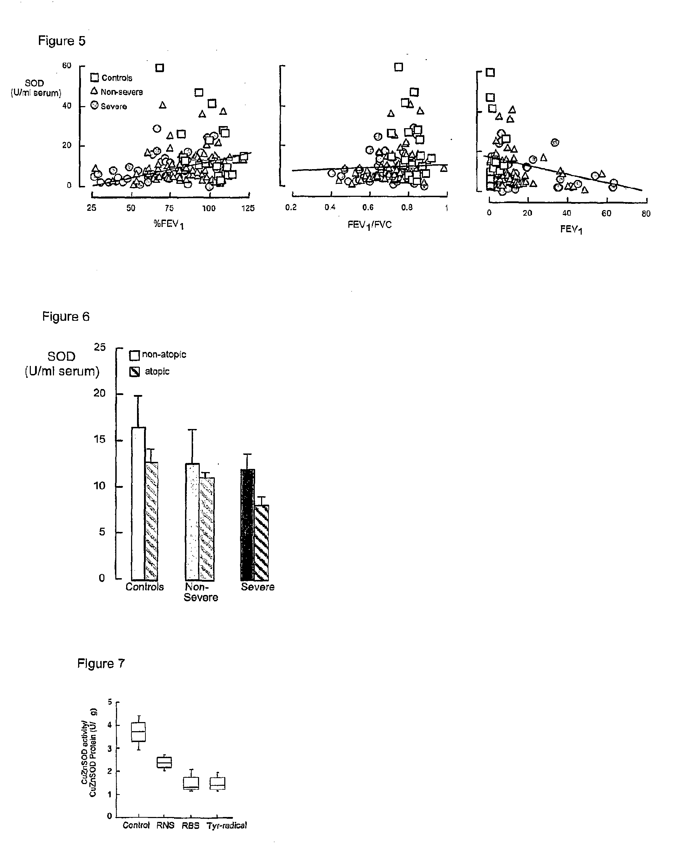

To investigate the relationship of antioxidants to lung functions, regression analyses were performed, with %FEV₁, FEV₁/FVC and the change in FEV₁ after bronchodilator (□FEV₁) (Figure 5). FEV₁% and □FEV₁ were most strongly correlated to SOD in the severe asthma group (Table 5), whereas no correlations were found with lung functions and other antioxidants (Table 6). These results suggest that serum SOD activity may serve as a global index of severity of asthma. Since half (19/40) of severe asthmatics could not undergo methacholine challenge testing due to an initial low FEV₁, SOD relation to PC20 could not be evaluated in severe asthma group. However, PC20 was positively correlated to SOD activity when all three groups were analyzed together (Table 3)

### Effect of corticosteroids on SOD response adjusted for age and gender

Severe asthmatics had greater steroid usage than non-severe asthmatics and healthy controls. Corticosteroids are related to improvement of airflow and restoration of airway SOD activity in non-severe asthmatics (De Raeve, H.R., et al., Am J Physiol 272(1 Pt 1):L148-54). Overall, corticosteroids taken orally, inhaled or injected did not have a clear influence on the SOD activity (p=0.506). Difference among airflow limitation groups (%FEV₁ <60, 60-80, >80) was also tested with an ANOVA model that adjusted for corticosteroid use. Despite the obvious relationship of corticosteroid usage to the groups, SOD activity (p=0.0012) was still significantly different among the groups when adjusted for corticosteroid use. When evaluating corticosteroid use, on the basis of method of administration (oral, inhaled or injected), only the use of injected corticosteroids may influence systemic measures of SOD activity in severe asthmatics (Table 7).

### Loss of SOD related to atopy

Although atopy is implicated in the cause of asthma, the relationship between antioxidant status and atopy has not been investigated. When comparing non-atopic versus atopic asthmatic subjects, atopic individuals were observed to have lower overall systemic levels of SOD activity (Figure 6) (p=0.027). These results are consistent with the possibility that allergen triggered inflammatory pathways may participate in loss of systemic SOD activity, perhaps through increasing oxidative stress (MacPherson, J.C., et al., J Immunol 166(9):5763-72; Wu, W., et al., J Clinc Invest 105(10):1455-63; Bowler, R.P., et al., J Allergy Clin Immunol 110(3):349-56), and subsequent inactivation of SOD.

### Mechanism of SOD inactivation

In the context of significant correlation of SOD activity to physiologic parameters of asthma and atopy, we investigated potential mechanisms of SOD inactivation in asthma. Previous *in vitro* studies indicate that SOD is exquisitely susceptible to oxidative modification and inactivation (Salo, D.C., et a., J Biol Chem 265(20):11919-27; Alvarez, B., et al., Free Radic Biol Med 37(6):813-22; Guo, W., et al., Am J Physiol Heart Circ Physiol 285(4):H1396-403; MacMillan-Crow, L.A., and J.A. Thompson, Arch Biochem Biophys 366(1):82-8; MacMillan-Crow, L.A., et al., Free Radic Biol Med 31(12):1603-8). Notably, eosinophil peroxidase-generated oxidants have been identified as specific participants in oxidative injury in both allergic and severe asthma, with 3-bromotyrosine (BrTyr), a specific protein modification generated by eosinophil peroxidase-catalyzed oxidation (MacPherson, J.C., et al., J Immunol 166(9):5763-72; Wu, W., et al., J Clinc Invest 105(10):1455-63; Wu, W., et al., J Biol Chem 274(36):25933-44; Wu, W., et al., Biochemistry 38(12):3538-48). CuZnSOD structure does not contain tyrosine residues, but oxidative modification of the enzyme may occur through effects on alternative susceptible target amino acids such as methionine, cysteine, histidine, tryptophan, arginine and lysine. CuZnSOD was therefore exposed to physiologically relevant levels of eosinophil peroxidase-generated reactive brominating species, reactive nitrogen species, or tyrosyl radicals to assess the potential role of oxidative pathways that might contribute to enzyme inactivation. All reactive species lead to loss of specific SOD activity (Figure 7). The magnitude of effect by reactive brominating species supports a potential role of eosinophil peroxidase-catalyzed inactivation *in vivo.* To test this hypothesis, plasma of asthmatic patients were analyzed by mass spectrometry to quantify levels of protein bound bromotyrosine, as a marker of eosinophil-derived reactive brominating species, dityrosine, an oxidative crosslink generated via a tyrosyl radical intermediate (Brennan, M.L., et al., J Biol Chem 277(20): 17415-27), and 3-nitrotyrosine, a stable protein modification generated by nitric oxide-derived oxidants (MacPherson, J.C., et al., J Immunol 166(9):5763-72). Remarkably, systemic levels of bromotyrosine demonstrated statistically significant inverse correlations with serum SOD activity (R=-0.404; p=0.049), consistent with loss of SOD activity as a consequence of reactive brominating species. Interestingly, no such relationship was observed with either nitrotyrosine or dityrosine, suggesting that neither NO-derived oxidants nor tyrosyl radical mediated oxidative crosslinks participate in SOD inactivation *in vivo.*

### DISCUSSION

The present study provides direct evidence to support global inhibition in systemic measures of SOD catalytic activity during asthma that are related to airflow limitation and asthma severity. The relationship of circulating SOD activity measures to plasma bromotyrosine levels, an oxidative modification characteristic of eosinophil peroxidase-generated brominating oxidants, is consistent with an oxidant mechanism of inactivation. The elevation of systemic total GSH levels observed in severe asthmatics may reflect the chronic oxidative stress experienced in asthmatics with severe airflow limitation. Together with the finding that atopic severe asthmatics have greatest loss of SOD activity, the present studies suggest that systemic measures of SOD inactivation may serve as a sensitive and quantitative functional measure of global oxidative and nitrative stress in asthma.

Loss of serum SOD activity in asthma may reflect a greater magnitude and/or ongoing systemic oxidative stress in severe asthma, with a consequent greater oxidative modification of SOD systemically. In support of this, the loss of SOD activity in serum of asthmatics is significantly lower when corrected for atopy, which is associated with systemic oxidant stress. Activated peripheral blood monocytes of atopic individuals produce superoxide when IgE binds to membrane receptors (Demoly, et al., J Allergy Clin Immunol 93(1 Pt 1):108-16) and serum eosinophil cationic protein (ECP), a biomarker of eosinophil activation, is increased with atopy and asthma severity (Joseph-Bowen, J., et al., J Allergy Clin Immunol 114(45):1040-5). While not wishing to be bound by theory, it is applicants' belief that lower serum SOD in asthma likely results from exposure to reactive oxidants, which may occur in the lung or systemically.

Reactive oxygen and nitrogen species can react with many amino acid targets including methionine, tyrosine, histidine, tryptophan, lysine and cysteine, profoundly altering the function of proteins by post-translational oxidative modification. All SOD enzymes are sensitive to oxidative modification and inactivation (Salo, D.C., et a., J Biol Chem 265(20):11919-27; Sharonov, B.P., et al., Biochem Biophys Res Commun 189(2):1129-35 Alvarez, B., et al., Free Radic Biol Med 37(6):813-22; Mamo, L.B., et al., Am J Respir Crit Care Med 170(3):313-8). *In vitro* studies have shown that ROS/RNS lead to oxidative and nitrative modification of tyrosine and inactivation of MnSOD and ECSOD, while Cu,ZnSOD can be inactivated by RNS through targeting of susceptible histidine residues (Alvarez, B., et al., Free Radic Biol Med 37(6):813-22; MacMillan-Crow, L.A., and J.A. Thompson, Arch Biochem Biophys 366(1):82-8; MacMillan-Crow, L.A., et al., Proc Natl Acad Sci U S A 93(21):11853-8). Recently, we have shown that oxidative modification/inactivation of MnSOD is present in asthmatic airway epithelial cells (Zheng, L., et al., J Clin Invest 114(4):529-41). Quantitative data on MnSOD oxidation/nitration in lungs of mild asthmatics with near-normal lung function shows that MnSOD tetramers possess at least 1 oxidative modification, which would lead to as much as 7% inactivation of MnSOD (Zheng, L., et al., J Clin Invest 114(4):529-41). Here, evidence consistent with SOD inactivation in the circulation due to oxidative/nitrative modifications is presented by the correlation of SOD activity with the levels of plasma bromotyrosine, an eosinophil-generated oxidative marker. Evidence consistent with a causative relationship between increased oxidants and SOD inactivation is supported by quantitative assay of CuZnSOD activity following exposures to eosinophil peroxidase-generated reactive species *in vitro* (Figure 7).

The present data show that serum eGPx protein and GPx activity are not upregulated in asthmatics as compared to controls, although prior studies demonstrate that eGPx is upregulated in asthmatic airway epithelial cells and in epithelial lining fluid ( Comhair, S.A., et al., Faseb J 15(1):70-78). The localized increase in lung eGPx but absence of increase in serum levels supports the concept that alterations in serum SOD levels may reflect systemic effects of oxidants.

It has been suggested that corticosteroids have a beneficial effect on antioxidants (De Raeve, H.R., et al., Am J Physiol 272(1 Pt 1):L148-54). Previous reports have shown that treatment with corticosteroid reduces oxidative stress and restores intracellular SOD activity levels in mild asthmatics (De Raeve, H.R., et al., Am J Physiol 272(1 Pt 1):L148-54; Majori, M., et al., Eur Respir J 11(1):133-8). In this study, history of treatment with inhaled or oral corticosteroids were not correlated with serum SOD activity measures in asthmatic subjects, but parenteral corticosteroid use was associated with SOD activity measures in asthmatic patients. Further studies can be conducted to determine if high dose systemic corticosteroids improve antioxidant responses in severe asthma.

Asthma is currently defined and diagnosed by a combination of clinical symptoms and physiologic abnormalities without well-controlled pathological and/or biological markers for severity. Measures of SOD activity, oxidative modification of proteins and/or GSH levels may serve as easily quantifiable circulating biomarkers to assess overall magnitude of oxidative stress, which the present studies reveal are related to severity and progression of asthma.

| **Table 4: Demographics, Pulmonary function and Corticosteroid usage for all subjects** | | | |
|---|---|---|---|
| | **Controls** | **Non-Severe Asthma** | **Severe Asthma** |
| **N** | 20 | 74 | 40 |
| **Mean age, yr** | 34.1 (2.7) | 33.3 (1.3) | 40.2 (2.2)* |
| **% FEV₁** | 100.4 (2.9) | 92.4 (2.2) | 65.4 (3.5) * |
| **% FVC** | 101.5 (10.8) | 84.7 (2.2) | 80.1 (3.4) * |
| **FEV₁/FVC** | 0.82 (0.01) | 0.77(0.1) | 0.67 (0.1) * |
| **Gender (F/M)** | 10/10 | 47/27 | 25/15 |
| **Race (A/AA/C/H/MR)** | 2/1/16/0/0/1 | 3/20/47/2/2 | 3/8/29/0/0 |
| **Sinusitus (%)** | 0/13 (0%) | 9/42 (17.3%) | 24/15 (61.5%) * |
| **Corticosteroids Inhaled (%)** | 0/13 (0%) | 18/61 (30%) | 37/40 (93%) * |
| **oral (%)** | 0/13 (0%) | 2/61 (3%) | 19/40 (48%)* |
| **injected (%)** | 0/13 (0%) | 1/61 (2%) | 4/40 (10%) * |
| **Atopy** | 6/13 (46%) | 54/62 (87%) | 29/40 (73%) * |
| **Smoking** | 0/13 | 0/74 | 0/40 |
| **Total cells x10⁶** | 10.2 (0.45) | 6.6 (0.25) | 7.8 (0.53) * |
| **%Neutrophils** | 56.3 (2.34) | 54.6 (1.7) | 59.3 (2.6) |
| **%Lymphocytes** | 33.9 (2.26) | 29.7 (1.27) | 31.5 (2.09) |
| **%Eosinophils** | 2.7 (0.31) | 3.8 (0.36) | 3.7 (0.49) |
| **%Basophils** | 0.93 (0.35) | 0.44 (0.06) | 2.7 (2.3) |
| **%Monocytes** | 7.5 (0.72) | 7.3 (0.29) | 5.9 (0.39) * |
| **IgE levels** | 58 (24) | 198 (30) | 463 (258) |

| | | | |
|---|---|---|---|
| Definition of abbreviations: F= female; M=male; A=Asian; AA=African American; C=Caucasian; H= Hispanic; MR=Multiple Race | | | |

Data are presented as means (SE), * p<0.05; Total cells and differentials are from whole blood.

| **Table 5: Correlations of total SOD activity with lung functions** | | | | |
|---|---|---|---|---|
| | **All groups** | **Controls** | **Non-Severe** | **Severe** |
| | | | | |
| **%FEV₁** | R=0.312 | R=-0.371 | R=0.240 | R=0.447 |
| | p<0.001 | p=0.105 | p=0.043 | p=0.004 |
| | | | | |
| **FEV₁/FVC** | R=0.296 | R=0.236 | R=0.338 | R=0.211 |
| | p<0.0001 | p=0.407 | p=0.004 | p=0.191 |
| | | | | |
| **□ FEV₁** | R=-0.334 | R=-0.245 | R=-0.243 | R=-0.449 |
| | p<0.001 | p=0.292 | p=0.040 | p=0.004 |

| | | | | |
|---|---|---|---|---|
| □FEV₁: % change in FEV₁ after 2 puffs of beta agonist inhaler | | | | |

| **Table 6: Correlations of serum antioxidants with lung functions in the 3 groups** | | | | |
|---|---|---|---|---|
| **Lung Functions** | **SOD** | **GSH** | **GPx** | **eGPx** |
| | (U/ml) | (□M) | (U/ml) | (ng/ml) |
| | | | | |
| **FEV₁** | R=0.295 | R=0.07 | R=-0.01 | R=0.157 |
| | p<0.001 | p=0.94 | p=0.94 | p=0.07 |
| | | | | |
| **%FEV₁** | R=0.312 | R=0.113 | R=-0.057 | R=0.124 |
| | p=0.001 | p=0.19 | p=0.51 | p=0.15 |
| | | | | |
| **%FVC** | R=0.113 | R=0.146 | R=-0.19 | R=0.122 |
| | p=0.2 | p=0.09 | p=0.022 | p=0.16 |
| **FEV₁/FVC** | R=0.296 | R=0.163 | R=-0.048 | R=0.01 |
| | p<0.001 | p=0.06 | p=0.58 | p=0.9 |
| | | | | |
| **□FEV₁** | R=-0.334 | R=-0.05 | R=-0.04 | R=0.01 |
| | p<0.001 | p=0.58 | p=0.63 | p=0.94 |
| | | | | |
| **PC20** | R=0.198 | R=0.081 | R=0.162 | R=-0.128 |
| | p=0.038 | p=0.4 | p=0.09 | p=0.18 |

**Table 7: Influence of Corticosteroid use on SOD activity**

| | Oral | | | Inhaled | | | Injected | | |
|---|---|---|---|---|---|---|---|---|---|
| | never | some/daily | p-value | never | some/daily | p-value | Never | some/daily | p-value |
| SOD (U/ml) | 10.2±0.9 | 11±2 | 0.684 | 11±2 | 10.2±0.9 | 0.506 | 9.8±0.8 | 20±5 | 0.021 |

| | | | | | | | | | |
|---|---|---|---|---|---|---|---|---|---|
| Some/daily: use at least 1 time/week to 2 times/day corticosteroid | | | | | | | | | |

## Claims

1. A method for identifying a subject who is at risk of having asthma or an analogous disease associated with high oxidative stress, high nitrative stress, or both at the disease site, comprising:
assaying for reduced levels of total superoxide dismutase (SOD) activity in a test sample of the subject, wherein the test sample is serum or plasma, and
wherein reduced levels of total SOD activity in the test sample as compared to levels of total SOD activity in a control sample indicates that the subject is at risk of having asthma, the analogous disease, or both.

2. The method of claim 1, wherein the subject has one or more symptoms associated with asthma, one or more physiologic parameters associated with asthma, or both.

3. The method of claim 2, wherein severity of the subject's asthma correlates with the extent of the reduction of total SOD activity levels in the test sample.

4. The method of claim 2, wherein levels of total SOD activity in the test sample are compared to levels of total SOD activity in corresponding samples from subjects lacking asthma.

5. The method of claim 1, wherein levels of total SOD activity in the test sample are compared to a baseline level of total SOD activity in a corresponding sample from the test subject.

6. The method of claim 1, wherein SOD activity is assayed employing a technique selected from UV, VIS, fluorescence spectrophotometry, or chemiluminescence.

7. A method of monitoring the progression of asthma in a subject, comprising determining levels of total SOD activity in a plurality of test samples over time, wherein the test samples are serum and plasma.

8. A method of evaluating the effect of an anti-inflammatory agent on a subject with asthma or an analogous disease associated with high oxidative stress or high nitrative stress or both at the disease site, comprising:
comparing levels of total SOD activity in serum or plasma of the subject following treatment of the subject with the anti-inflammatory drug to levels of total SOD activity in the blood, serum, or plasma of the subject prior to treatment with the antiinflammatory agent, and/or
comparing levels of total SOD activity in the serum or plasma of the subject following treatment with the anti-inflammatory agent with a control value based on levels of total SOD activity in the serum or plasma, respectively, of a control subject.

9. A method of diagnosing asthma or an analogous diseases associated with high nitrative stress, or high oxidative stress, or both at the disease site in a subject, comprising:
assaying for elevated levels of one or more oxidatively-modified superoxide dismutase (SOD) species selected from extracellular (EC)-SOD, CuZn SOD, and MnSOD, or any combination thereof in a test sample of the subject;
wherein the test sample is serum or plasma; and
wherein the presence of elevated levels of said one or more oxidatively-modified SOD species in the test sample as compared to levels of the one or more oxidatively-modified SOD species in a control sample indicates that the subject is at risk of having asthma, the analogous disease, or both.

10. The method of claim 9, wherein the subject has one or more symptoms associated with asthma, one or more physiologic parameters associated with ashthma, or both, and wherein the extent of elevation in levels of said one or more oxidatively-modified SOD species in the sample correlates with the severity of the subject's asthma.

11. The method of claim 10, further comprising the step of comparing levels of the one or more oxidatively-modified SOD mass species in the test sample to levels of the one or more oxidatively-modified SOD species in corresponding samples from normal subjects lacking asthma.

12. The method of claim 9, wherein levels of the one or more oxidiatively-modified SOD species in the test sample are compared to a baseline level of the one or more oxidatively- modified SOD species in a corresponding sample from the test subject.

13. The method of claim 10, wherein levels of the one or more oxidatively-modified SOD species are compared to an internal standard based on total levels of the one or more SOD species in the test sample, or based on the levels of the one or more unmodified SOD species in the test sample.

14. The method of claim 9, wherein levels of the one or more oxidatively-modified SOD species are assayed by contacting the sample with a binding reagent specific for the one or more oxidatively-modified SOD species generated by exposure of the one or more SOD species to an eosinophil peroxidase (EPO)-H₂O₂-NO₂- system, an EPO-H₂O₂-Br system, HOBr, ONOO⁻, an EPO-H₂O₂-tyrosine system, a myeloperoxidase (MPO) -H₂O₂-NO₂- system, an MPO-H₂O₂-Cl⁻ system, an MPO-H₂O₂-tyrosine system, HOCI, or to copper or iron (+/- H₂O₂) catalyzed oxidation, and assaying for the formation of a complex between the binding reagent and a protein or peptide in said sample.

15. The method of claim 9, wherein the one or more oxidatively modified SOD species comprises one or more of the following modifications: a modified porphyrin prosthetic group, a bromotyrosine, a dibromotyrosine, a nitrotyrosine, a chlorotyrosine, a dichlorotyrosine, a methionine sulfoxide, cysteic acid, sulfenic acid, a carbonyl, a homocitrulline, an amino adipoic acid, cystine, a dihydroxyphenylalanine, a dityrosine, an ortho-tyrosine, and a meta-tyrosine.

16. A method of monitoring the progression of asthma in a subj ect, comprising determining levels of one or more oxidatively-modified SOD species in a plurality of test samples of the subject over time,
wherein the test samples are serum and plasma.

17. A method of evaluating the effect of an anti-inflammatory agent on a subject with asthma or an analogous disease associated with high oxidative stress or high nitrative stress or both at the disease site, comprising:
comparing levels of one or more oxidatively-modified SOD species in serum or plasma of the subject following treatment of the subject with the anti-inflammatory drug to levels of the one or more oxidatively-modified SOD species in the serum or plasma of the subject prior to treatment with the anti-inflammatory agent, and/or
comparing levels of the one or more oxidatively-modified SOD species in the serum or plasma of the subject following treatment with the anti-inflammatory agent with a control value based on levels of the one or more oxidatively-modified SOD species in the serum or plasma, respectively, of a control subject.

18. A diagnostic kit for diagnosing asthma, or an analogous disease associated with high oxidative and nitrative stress at the disease site or both, said kit comprising one or more binding reagents that specifically bind to an oxidatively-modified form of an SOD species.

19. The diagnostic kit of claim 18, further comprising instructions for using the binding reagent to diagnose asthma or the analogous disease or both, or for using the binding reagent to assess the severity of asthma or the analogous disease in the test subject, or both.

20. The diagnostic kit of claim 18, wherein said binding agent is a monoclonal or polyclonal antibody, a fragment or derivative thereof, and wherein the one or more oxidatively-modified SOD species for making the antibody are generated by exposure of the one or more SOD species to an eosinophil peroxidase (EPO)-H₂O₂-NO₂- system, an EPO-H₂O₂-Br system, HOBr, ONOO⁻ an EPO-H₂O₂-tyrosine system, a myeloperoxidase (MPO) -H₂O₂-NO₂-system, an MPO-H₂O₂-Cl⁻ system, an MPO-H₂O₂-tyrosine system, HOCI, or copper or iron (+/- H₂O₂) catalyzed oxidation.

21. The diagnostic kit of claim 18, wherein the binding reagent specifically binds to an oxidatively-modified SOD species comprising one or more of the following modifications: a modified porphyrin prosthetic group, a bromotyrosine, a dibromotyrosine, a nitrotyrosine, a chlorotyrosine, a dichlorotyrosine, a methionine sulfoxide, cysteic acid, sulfenic acid, a carbonyl, a homocitrulline, an amino adipoic acid, cystine, a dihydroxyphenylalanine, a dityrosine, an ortho-tyrosine, and a meta-tyrosine.

## Patentansprüche

1. Verfahren zur Identifizierung eines Individuums, bei dem das Risiko von Asthma oder einer ähnlichen Erkrankung in Zusammenhang mit hohem oxidativem Stress, hohem nitrativem Stress oder beidem an der Stelle der Erkrankung besteht, wobei das Verfahren Folgendes umfasst:
das Durchführen von Tests in Bezug auf reduzierte Gesamt-Superoxiddismutase- (SOD-) Aktivität in einer Probe von dem Individuum, wobei die Probe Serum oder Plasma ist, und
worin eine reduzierte Gesamt-SOD-Aktivität in der Probe im Vergleich zu der Gesamt-SOD-Aktivität in einer Kontrollprobe angibt, dass bei dem Individuum das Risiko besteht, dass es an Asthma, einer ähnlichen Erkrankung oder beiden erkrankt.

2. Verfahren nach Anspruch 1, worin das Individuum ein oder mehrere Symptome, die in Zusammenhang mit Asthma auftreten, ein oder mehrere physiologische Parameter in Zusammenhang mit Asthma oder beides aufweist.

3. Verfahren nach Anspruch 2, worin die Schwere der Asthmaerkrankung des Individuums mit dem Ausmaß der Reduktion der Gesamt-SOD-Aktivität in der Probe korreliert.

4. Verfahren nach Anspruch 2, worin die Gesamt-SOD-Aktivität in der Probe mit der Gesamt-SOD-Aktivität in entsprechenden Proben von Individuen ohne Asthma verglichen wird.

5. Verfahren nach Anspruch 1, worin die Gesamt-SOD-Aktivität in der Probe mit einem Grundlinienwert der Gesamt-SOD-Aktivität in einer entsprechenden Probe des Testindividuums verglichen wird.

6. Verfahren nach Anspruch 1, worin die SOD-Aktivität unter Anwendung eines Verfahrens getestet wird, das aus UV-, VIS-, Fluoreszenzspektrophotometrie oder Chemilumineszenz ausgewählt ist.

7. Verfahren zur Überwachung des Fortschreitens von Asthma in einem Individuum, das die Bestimmung der Gesamt-SOD-Aktivität in einer Vielzahl von Proben im Lauf der Zeit umfasst, wobei die Proben Serum und Plasma sind.

8. Verfahren zur Bewertung der Wirkung eines entzündungshemmenden Mittels auf ein Individuum mit Asthma oder einer ähnlichen Erkrankung in Zusammenhang mit hohem oxidativem Stress oder hohem nitrativem Stress oder beidem an der Stelle der Erkrankung, wobei das Verfahren Folgendes umfasst:
den Vergleich der Gesamt-SOD-Aktivität in Serum oder Plasma des Individuums nach Behandlung des Individuums mit dem entzündungshemmenden Arzneimittel mit der Gesamt-SOD-Aktivität in Blut, Serum oder Plasma des Individuums vor der Behandlung mit dem entzündungshemmenden Mittel und/oder
den Vergleich der Gesamt-SOD-Aktivität in Serum oder Plasma des Individuums nach Behandlung mit dem entzündungshemmenden Mittel mit einem Kontrollwert auf Grundlage der Gesamt-SOD-Aktivität in Serum bzw. Plasma eines Kontrollindividuums.

9. Verfahren zur Diagnose von Asthma oder ähnlichen Erkrankungen in Zusammenhang mit hohem oxidativem Stress oder hohem nitrativem Stress oder beidem an der Stelle der Erkrankung, wobei das Verfahren Folgendes umfasst:
das Durchführen von Tests in Bezug auf erhöhte Spiegel einer oder mehrere oxidativ modifizierter Superoxiddismutase- (SOD-) Spezies, ausgewählt aus extrazellulärer (EC-) SOD, CuZn-SOD und MnSOD oder einer beliebigen Kombination davon, in einer Probe von dem Individuum;
worin die Probe Serum oder Plasma ist und
worin das Vorliegen erhöhter Spiegel einer oder mehrerer oxidativ modifizierter SOD-Spezies in der Probe im Vergleich zu den Spiegeln der einen oder mehreren oxidativ modifizierten SOD-Spezies in einer Kontrollprobe angibt, dass bei dem Individuum das Risiko besteht, an Asthma, einer ähnlichen Erkrankung oder beiden zu leiden.

10. Verfahren nach Anspruch 9, worin das Individuum ein oder mehrere Symptome, die in Zusammenhang mit Asthma auftreten, ein oder mehrere physiologische Parameter in Zusammenhang mit Asthma oder beides aufweist und worin das Ausmaß der erhöhten Spiegel der einen oder mehreren oxidativ modifizierten SOD-Spezies in der Probe mit der Schwere der Asthmaerkrankung des Individuums korreliert.

11. Verfahren nach Anspruch 10, das ferner den Schritt des Vergleichens der Spiegel einer oder mehrerer oxidativ modifizierter SOD-Massenspezies in der Probe mit den Spiegeln einer oder mehrerer oxidativ modifizierter SOD-Spezies in entsprechenden Proben von normalen Individuum ohne Asthma umfasst.

12. Verfahren nach Anspruch 9, worin Spiegel der einen oder mehreren oxidativ modifizierten SOD-Spezies in der Probe mit Grundlinienwerten der einen oder mehreren oxidativ modifizierten SOD-Spezies in einer entsprechenden Probe von dem getesteten Individuum verglichen werden.

13. Verfahren nach Anspruch 10, worin Spiegel der einen oder mehreren oxidativ modifizierten SOD-Spezies mit einem inneren Standard auf Grundlage von Gesamtwerten der einen oder mehreren SOD-Spezies in der Probe oder auf Grundlage einen oder mehreren nicht modifizierten SOD-Spezies in der Probe verglichen werden.

14. Verfahren nach Anspruch 9, worin Spiegel der einen oder mehreren oxidativ modifizierten SOD-Spezies getestet werden, indem die Probe mit einem Bindungsreagens kontaktiert wird, das spezifisch für die eine oder mehreren oxidativ modifizierten SOD-Spezies ist und durch das Aussetzen der einen oder mehreren SOD-Spezies gegenüber einem EPO- (eosinophile-Peroxidase-) H₂O₂-NO₂-System, einem EPO-H₂O₂-Br-System, HOBr, ONOO⁻, einem EPO-H₂O₂-Tyrosinsystem, einem MPO- (Myeloperoxidase-) H₂O₂-NO₂-System, einem MPO-H₂O₂-Cl⁻-System, einem MPO-H₂O₂-Tyrosin-System, HOCI oder einer durch Kupfer oder Eisen (+/- H₂O₂) katalysierten Oxidation hergestellt wird, und ein Test in Bezug auf die Bildung eines Komplexes zwischen dem Bindungsreagens und einem Protein oder Peptid in der Probe durchgeführt wird.

15. Verfahren nach Anspruch 9, worin die eine oder mehreren oxidativ modifizierten SOD-Spezies eine oder mehrere der folgenden Modifikationen umfassen: eine modifizierte Porphyrin-Prosthesegruppe, ein Bromtyrosin, ein Dibromtyrosin, ein Nitrotyrosin, ein Chlortyrosin, ein Dichlortyrosin, ein Methioninsulfoxid, Cysteinsäure, Sulfensäure, ein Carbonyl, ein Homocitrullin, eine Aminoadipinsäure, Cystin, ein Dihydroxyphenylalanin, ein Dityrosin, ein ortho-Tyrosin und ein meta-Tyrosin.

16. Verfahren zur Überwachung des Fortschreitens von Asthma in einem Individuum, das die Bestimmung der Spiegel einer oder mehrerer oxidativ modifizierter SOD-Spezies in einer Vielzahl von Testproben des Individuums im Lauf der Zeit umfasst, worin die Testproben Serum und Plasma sind.

17. Verfahren zur Bewertung der Wirkung eines entzündungshemmenden Mittels auf ein Individuum mit Asthma oder einer ähnlichen Erkrankung in Zusammenhang mit hohem oxidativem Stress oder hohem nitrativem Stress oder beidem an der Stelle der Erkrankung, wobei das Verfahren Folgendes umfasst:
den Vergleich der einen oder mehreren oxidativ modifizierten SOD-Spezies in Serum oder Plasma des Individuums nach Behandlung des Individuums mit dem entzündungshemmenden Arzneimittel mit der einen oder mehreren oxidativ modifizierten SOD-Spezies in Blut, Serum oder Plasma des Individuums vor der Behandlung mit dem entzündungshemmenden Mittel und/oder
den Vergleich der einen oder mehreren oxidativ modifizierten SOD-Spezies in Serum oder Plasma des Individuums nach Behandlung mit dem entzündungshemmenden Mittel mit einem Kontrollwert auf Grundlage der der einen oder mehreren oxidativ modifizierten SOD-Spezies in Serum bzw. Plasma eines Kontrollindividuums.

18. Diagnoseset für die Diagnose von Asthma oder einer ähnlichen Erkrankung in Zusammenhang mit hohem oxidativem Stress oder hohem nitrativem Stress oder beidem an der Stelle der Erkrankung, wobei das Set ein oder mehrere Bindungsreagenzien umfasst, die spezifisch an eine oxidativ modifizierte Form einer SOD-Spezies binden.

19. Diagnoseset nach Anspruch 18, das ferner Anweisungen zur Anwendung des Bindungsreagens zur Diagnose von Asthma oder der ähnlichen Erkrankung oder beiden oder zur Anwendung des Bindungsreagens zur Bewertung der Schwere der Asthmaerkrankung oder der ähnlichen Erkrankung in dem Testindividuum oder für beides umfasst.

20. Diagnoseset nach Anspruch 18, worin das Bindungsmittel ein monoklonaler oder polyklonaler Antikörper, ein Fragment oder Derivat davon ist und worin die eine oder mehreren oxidativ modifizierten SOD-Spezies zur Herstellung des Antikörpers durch das Aussetzen der einen oder mehreren SOD-Spezies gegenüber einem EPO-(eosinophile-Peroxidase-) H₂O₂-NO₂-System, einem EPO-H₂O₂-Br⁻-System, HOBr, ONOO⁻, einem EPO-H₂O₂-Tyrosinsystem, einem MPO- (Myeloperoxidase-) H₂O₂-NO₂-System, einem MPO-H₂O₂-Cl⁻-System, einem MPO-H₂O₂-Tyrosin-System, HOCI oder einer durch Kupfer oder Eisen (+/- H₂O₂) katalysierten Oxidation hergestellt werden.

21. Diagnoseset nach Anspruch 18, worin das Bindungsreagens spezifisch an eine oxidativ modifizierte SOD-Spezies bindet, die eine oder mehrere der folgenden Modifikationen aufweist: eine modifizierte Porphyrin-Prosthesegruppe, ein Bromtyrosin, ein Dibromtyrosin, ein Nitrotyrosin, ein Chlortyrosin, ein Dichlortyrosin, ein Methioninsulfoxid, Cysteinsäure, Sulfensäure, ein Carbonyl, ein Homocitrullin, eine Aminoadipinsäure, Cystin, ein Dihydroxyphenylalanin, ein Dityrosin, ein ortho-Tyrosin und ein meta-Tyrosin.

## Revendications

1. Procédé d'identification d'un sujet qui présente un risque de souffrir d'asthme ou d'une maladie analogue associée à un stress oxydatif élevé, à un stress nitratif élevé ou aux deux au niveau du site de la maladie, comprenant :
l'analyse de niveaux réduits de l'activité des superoxyde dismutases (SOD) totales dans un échantillon d'essai du sujet,
dans lequel l'échantillon d'essai est du sérum ou du plasma, et
dans lequel la présence de niveaux réduits d'activité des SOD totales dans l'échantillon d'essai par rapport aux niveaux d'activité des SOD totales dans un échantillon témoin indique que le sujet présente un risque de souffrir d'asthme, de la maladie analogue ou des deux.

2. Procédé selon la revendication 1, dans lequel le sujet présente un ou plusieurs symptômes associés à l'asthme, un ou plusieurs paramètres physiologiques associés à l'asthme ou les deux.

3. Procédé selon la revendication 2, dans lequel la sévérité de l'asthme du sujet est en corrélation avec l'ampleur de la réduction des niveaux d'activité des SOD totales dans l'échantillon d'essai.

4. Procédé selon la revendication 2, dans lequel les niveaux d'activité des SOD totales dans l'échantillon d'essai sont comparés aux niveaux d'activité des SOD totales dans des échantillons correspondants provenant de sujets ne souffrant pas d'asthme.

5. Procédé selon la revendication 1, dans lequel les niveaux d'activité des SOD totales dans l'échantillon d'essai sont comparés à un niveau de base de l'activité des SOD totales dans un échantillon correspondant provenant du sujet soumis à l'essai.

6. Procédé selon la revendication 1, dans lequel l'activité de la SOD est analysée en employant une technique choisie parmi la spectrophotométrie UV, VIS, de fluorescence, ou la chimioluminescence.

7. Procédé de surveillance de la progression de l'asthme chez un sujet, comprenant la détermination des niveaux d'activité des SOD totales dans une pluralité d'échantillons d'essai au cours du temps, dans lequel les échantillons d'essai sont du sérum et du plasma.

8. Procédé d'évaluation de l'effet d'un anti-inflammatoire sur un sujet souffrant d'asthme ou d'une maladie analogue associée à un stress oxydatif élevé ou à un stress nitratif élevé ou aux deux au niveau du site de la maladie, comprenant :
la comparaison des niveaux d'activité des SOD totales dans le sérum ou le plasma du sujet après le traitement du sujet par l'anti-inflammatoire aux niveaux d'activité des SOD totales dans le sang, le sérum ou le plasma du sujet avant le traitement par l'anti-inflammatoire, et/or
la comparaison des niveaux d'activité des SOD totales dans le sérum ou le plasma du sujet après le traitement par l'anti-inflammatoire à une valeur témoin basée sur les niveaux d'activité des SOD totales dans le sérum ou le plasma, respectivement, d'un sujet témoin.

9. Procédé de diagnostic de l'asthme ou de maladies analogues associées à un stress nitratif élevé ou à un stress oxydatif élevé ou aux deux au niveau du site de la maladie chez un sujet, comprenant :
l'analyse de niveaux élevés d'une ou plusieurs espèces de superoxyde dismutases (SOD) modifiées sur le plan oxydatif choisies parmi la (EC)-SOD extracellulaire, la CuZn SOD et la MnSOD ou toute combinaison de celles-ci dans un échantillon d'essai du sujet ;
dans lequel l'échantillon d'essai est du sérum ou du plasma ; et
dans lequel la présence de niveaux élevés de ladite (desdites) une ou plusieurs espèces de SOD modifiées sur le plan oxydatif dans l'échantillon d'essai par rapport aux niveaux de l'une ou plusieurs espèces de SOD modifiées sur le plan oxydatif dans un échantillon témoin indique que le sujet présente un risque de souffrir d'asthme, de la maladie analogue ou des deux.

10. Procédé selon la revendication 9, dans lequel le sujet présente un ou plusieurs symptômes associés à l'asthme, un ou plusieurs paramètres physiologiques associés à l'asthme ou les deux, et dans lequel l'ampleur de l'élévation des niveaux de ladite (desdites) une ou plusieurs espèces de SOD modifiées sur le plan oxydatif dans l'échantillon est en corrélation avec la sévérité de l'asthme du sujet.

11. Procédé selon la revendication 10, comprenant en outre l'étape de comparaison des niveaux de la ou des espèces de SOD modifiées dans l'échantillon d'essai aux niveaux de la ou des espèces de SOD modifiées sur le plan oxydatif dans des échantillons correspondants provenant de sujets normaux ne souffrant pas d'asthme.

12. Procédé selon la revendication 9, dans lequel les niveaux de la ou des espèces de SOD modifiées dans l'échantillon d'essai sont comparés à un niveau de base de la ou des espèces de SOD modifiées dans un échantillon correspondant provenant du sujet soumis à l'essai.

13. Procédé selon la revendication 10, dans lequel les niveaux de la ou des espèces de SOD modifiées sur le plan oxydatif sont comparés à un standard interne basé sur les niveaux totaux de la ou des espèces de SOD dans l'échantillon d'essai, ou basé sur les niveaux de la ou des espèces de SOD non modifiées dans l'échantillon d'essai.

14. Procédé selon la revendication 9, dans lequel les niveaux de la ou des espèces de SOD modifiées sur le plan oxydatif sont déterminés en mettant en contact l'échantillon avec un réactif de liaison spécifique à la ou aux espèces de SOD modifiées sur le plan oxydatif générées par exposition de la ou des espèces de SOD à une oxydation catalysée par un système éosinophile peroxydase (EPO)-H₂O₂-NO₂-, un système EPO-H₂O₂-Br⁻, HOBr, ONOO⁻, un système EPO-H₂O₂-tyrosine, un système myéloperoxydase (MPO)-H₂O₂-NO₂-, un système MPO-H₂O₂-Cl⁻, un système MPO-H₂O₂-tyrosine, HOCI, ou du cuivre ou du fer (+/-H₂O₂), et l'analyse de la formation d'un complexe entre le réactif de liaison et une protéine ou un peptide dans ledit échantillon.

15. Procédé selon la revendication 9, dans lequel la ou les espèces de SOD modifiées sur le plan oxydatif comprennent une ou plusieurs des modifications suivantes : un groupe prosthétique porphyrine modifié, une bromotyrosine, une dibromotyrosine, une nitrotyrosine, une chlorotyrosine, une dichlorotyrosine, un sulfoxyde de méthionine, de l'acide cystéique, de l'acide sulfénique, un carbonyle, une homocitrulline, un acide amino-adipoïque, une cystine, une dihydroxyphénylalanine, une dityrosine, une ortho-tyrosine et une méta-tyrosine.

16. Procédé de surveillance de la progression de l'asthme chez un sujet, comprenant la détermination des niveaux d'une ou plusieurs espèces de SOD modifiées sur le plan oxydatif dans une pluralité d'échantillons d'essai du sujet au cours du temps,
dans lequel les échantillons d'essai sont du sérum et plasma.

17. Procédé d'évaluation de l'effet d'un anti-inflammatoire sur un sujet souffrant d'asthme ou d'une maladie analogue associée à un stress oxydatif élevé ou à un stress nitratif élevé ou aux deux au niveau du site de la maladie, comprenant :
la comparaison des niveaux d'une ou plusieurs espèces de SOD modifiées sur le plan oxydatif dans le sérum ou le plasma du sujet après le traitement du sujet par l'anti-inflammatoire aux niveaux de la ou des espèces de SOD modifiées sur le plan oxydatif dans le sérum ou le plasma du sujet avant le traitement par l'anti-inflammatoire, et/ou
la comparaison des niveaux de la ou des espèces de SOD modifiées sur le plan oxydatif dans le sérum ou le plasma du sujet après le traitement par l'anti-inflammatoire à une valeur témoin basée sur les niveaux de la ou des espèces de SOD modifiées sur le plan oxydatif dans le sérum ou le plasma, respectivement, d'un sujet témoin.

18. Kit de diagnostic servant à diagnostiquer l'asthme ou une maladie analogue associée à un stress oxydatif et nitratif élevé au niveau du site de la maladie ou aux deux, ledit kit comprenant un ou plusieurs réactifs de liaison qui se lient spécifiquement à une forme modifiée sur le plan oxydatif d'une espèce de SOD.

19. Kit de diagnostic selon la revendication 18, comprenant en outre des instructions d'utilisation du réactif de liaison pour diagnostique l'asthme ou la maladie analogue ou les deux, ou d'utilisation du réactif de liaison pour évaluer la sévérité de l'asthme ou de la maladie analogue chez le sujet soumis à l'essai, ou les deux.

20. Kit de diagnostic selon la revendication 18, dans lequel ledit agent de liaison est un anticorps monoclonal ou polyclonal, un fragment ou un dérivé de celui-ci, et dans lequel la ou les espèces de SOD modifiées sur le plan oxydatif servant à marquer l'anticorps sont générées par exposition de la ou des espèces de SOD à une oxydation catalysée par un système éosinophile peroxydase (EPO)-H₂O₂-NO₂-, un système EPO-H₂O₂-Br⁻, HOBr, ONOO⁻, un système EPO-H₂O₂-tyrosine, un système myéloperoxydase (MPO)-H₂O₂-NO₂-, un système MPO-H₂O₂-Cl⁻, un système MPO-H₂O₂-tyrosine, HOCI, ou du cuivre ou du fer (+/-H₂O₂).

21. Kit de diagnostic selon la revendication 18, dans lequel le réactif de liaison se lie spécifiquement à une espèce de SOD modifiée sur le plan oxydatif comprenant une ou plusieurs des modifications suivantes : un groupe prosthétique porphyrine modifié, une bromotyrosine, une dibromotyrosine, une nitrotyrosine, une chlorotyrosine, une dichlorotyrosine, un sulfoxyde de méthionine, de l'acide cystéique, de l'acide sulfénique, un carbonyle, une homocitrulline, un acide amino-adipoïque, une cystine, une dihydroxyphénylalanine, une dityrosine, une ortho-tyrosine et une méta-tyrosine.
